# EUROPÄISCHE PATENTSCHRIFT

(11) **EP 2 288 391 B1**
(45) Veröffentlichungstag und Bekanntmachung des Hinweises auf die Patenterteilung: **10.12.2014**
(21) Anmeldenummer: 09733006.2
(22) Anmeldetag: 14.04.2009
(51) Int. Cl.: A61M 1/10

(54) **VORRICHTUNG UND SYSTEM ZUR UNTERSTÜTZUNG UND/ODER ÜBERNAHME DER PUMPFUNKTION DES HERZENS**
DEVICE AND SYSTEM FOR SUPPORTING AND/OR TAKE-OVER OF THE PUMPING FUNCTION OF THE HEART
DISPOSITIF ET SYSTÈME DESTINÉS À ASSISTER ET/OU PRENDRE EN CHARGE LA FONCTION DE POMPAGE DU COEUR

(30) Priorität: 14.04.2008 DE 102008018919
(43) Veröffentlichungstag der Anmeldung: 02.03.2011
(73) Patentinhaber: PPA Technologies Gmbh, 07745 Jena (DE)
(72) Erfinder: OTTO, Thomas, 07745 Jena (DE); KLEIN, Andreas, 07745 Jena (DE)
(74) Vertreter: Von Rohr Patentanwälte Partnerschaft mbB
(86) Internationale Anmeldenummer: PCT/EP2009/002710
(87) Internationale Veröffentlichungsnummer: WO 2009/127384

(56) Entgegenhaltungen:
- WO-A1-2005/110513
- WO-A2-2007/062239
- US-A1- 2001 041 821
- US-A1- 2004 102 674

## Beschreibung

Die Erfindung betrifft eine Vorrichtung nach dem Oberbegriff des Anspruchs 1 sowie ein System mit einer Vorrichtung der vorgenannten Art.

Eine Vorrichtung mit den Merkmalen der eingangs genannten Art ist bereits aus der US 2004/102674 A1 und der WO 2007/062239 A2 bekannt.

Eine weitere Vorrichtung zur perikardialen Unterstützung und/oder Übernahme der Herztätigkeit der vorgenannten Art ist beispielsweise aus der Druckschrift DE 199 51 220 A1 bekannt. Es handelt sich dabei um ein wenig invasives, d.h. perkutan implantierbares System, zur mechanischen Unterstützung und zum temporären Ersatz der Pumpfunktion des Herzens. Die Vorrichtung wird nach Sondierung des Herzbeutels perkutan in den Herzbeutel eingebracht oder am Ende einer Operation in den Herzbeutel chirurgisch positioniert und dort mit einer Doppelmembran um die rechte und linke Herzkammer gelegt. Dabei ist die Vorrichtung in deflatiertem Zustand der Doppelmembran so dünn, daß eine Kompression der Nachbarorgane vermieden wird. Nach der Implantation wird der Hohlraum der Doppelmembran über einen Verbindungsschlauch rhythmisch mit einem Fluid beaufschlagt, welches entweder ein Gas (Helium oder CO₂) oder eine geeignete Flüssigkeit sein kann. Durch dieses rhythmische Inflatieren und Deflatieren des Hohlraums der Doppelmembran und weil die äußere Membran im Gegensatz zur inneren Membran nicht dehnbar ist, kommt es zu einer Druckübertragung und Kompression des Herzens über die das Herz umschließende Doppelmembran. Dabei wird das Blut aus der rechten Herzkammer in die Pulmonalarterie und gleichzeitig aus der linken Kammer in die Aorta ausgetrieben oder bei vorhandener Pumpfunktion des Herzens die systolische Auswurfarbeit des Herzmuskels unterstützt.

Eine ähnliche Vorrichtung - allerdings epikardial arbeitend - ist aus der WO 2005/110513 A1 bekannt. Diese Vorrichtung sieht ebenfalls eine Doppelmembran zur epikardialen Unterstützung und/oder Übernahme der Herztätigkeit vor, die eine elastische innere Membran und eine nicht-dehnbare äußere Membran sowie einen dazwischen gebildeten, geschlossenen und mittels eines Fluids inflatierbaren und deflatierbaren Hohlraum mit einer dem rechten Ventrikel zugeordneten ersten Kammer und einer dem linken Ventrikel zugeordneten zweiten Kammer aufweist. Die erste Kammer und die zweite Kammer sind über wenigstens ein Ventil in einer die beiden Kammern separierenden Trennwand miteinander verbunden. Es können je nach Bedarf Doppelmembranen hergestellt werden, welche die alleinige Augmentation des rechten Ventrikels oder - in einer anderen Ausführungsform - nur des linken Ventrikels, beides unter Beibehaltung der Möglichkeit einer Unterstützung beider Ventrikel gleichzeitig, ermöglichen.

Die zuvor beschriebenen Vorrichtungen werden direkt am Herzen oder an den das Herz im Brustkorb umgebenden Organen befestigt, so daß die Vorrichtungen stets in unmittelbarem Kontakt mit der Herzoberfläche stehen. Das Anbringen und Fixieren der Vorrichtungen auf dem weichen empfindlichen Gewebe von Herz, Lungen und großen Gefäßen ist jedoch aufgrund der makro- und mikroanatomischen Beschaffenheit der Organe nur beschränkt möglich. Auftretende Reibungskräfte zwischen der Vorrichtung und dem Gewebe können bereits sehr kurzfristig, innerhalb von Stunden und Tagen, zu erheblichen irreparablen Gewebeschädigungen führen, Penetrationen der Herzkammern und von großen Gefäßen im Brustkorb führen innerhalb von Minuten zum Tod des Patienten. Im übrigen kann es bei Anbringen und Fixieren der Vorrichtungen direkt am Herzen zu einer Behinderung bzw. Beeinträchtigung der vorhandenen Herzeigenaktion kommen, wobei das Herz aufgrund seiner mechanischen Pumpfunktion eine zyklische Verkürzung und Verlängerung in der Herzachse bei gleichzeitiger spiralartiger Drehung um die Herzachse zeigt. Eine weitere Herausforderung für in den Brustkorb einzubringende Vorrichtungen stellen die Raum- und Lageverhältnisse zwischen den Organen (Herz, Lunge, große Gefäße) einerseits sowie zwischen den Brustkorborganen und der Innenwand des Brustkorbes andererseits dar. Sie bieten sehr eingeschränkte Möglichkeiten, in den Brustkorb eingebrachte Instrumente oder Vorrichtungen am bzw. um das Herz zu entfalten bzw. das Herz zu umfahren.

Aufgabe der vorliegenden Erfindung ist es, eine Vorrichtung und ein System jeweils der eingangs genannten Art zur Verfügung zu stellen, mit denen im Bedarfsfall eine direkte Herzdruckmassage bzw. eine gerichtete zyklische Kompression und Dekompression des Herzens ermöglicht wird, wodurch die mechanische Pumpfunktion des Herzens unterstützt bzw. ersetzt werden kann. Im übrigen soll die Vorrichtung in einfacher Weise minimal invasiv in die Brusthöhle einbringbar und entfernbar sein, d.h. ohne konventionelle chirurgische Eröffnung des Brustkorbes (Thorakotomie). Der Aufbau und die Funktionsweise der Vorrichtung sollen darüber hinaus eine lagegetreue Positionierung innerhalb der Brusthöhle am bzw. um das Herz ermöglichen, ohne daß eine Schädigung des Herzens und/oder der umliegenden Organe befürchtet werden muß. Im Funktionszustand, d.h. bei zyklischer Kompression und Dekompression des Herzens, soll die Beweglichkeit des schlagenden Herzens nicht oder nur in vernachlässigbarem Maße beeinträchtigt sein. Schließlich sollen die Vorrichtung und das System kostengünstig herstellbar sein.

Zur Lösung der vorgenannten Aufgaben wird eine Vorrichtung mit den Merkmalen von Anspruch 1 vorgeschlagen.

Um die Formanpassung des Ummantelungsteils an das Herz beim Überführen vom Einführ- in den Funktionszustand zu bewerkstelligen, ist vorgesehen, dass der Ummantelungsteil mit Bezug auf den Einführzustand zwei sich in Längsrichtung erstreckende aufgerollte und/oder gefaltete inflatierbare Randabschnitte aufweist, wobei ein Basisteil zwischen den Randabschnitten angeordnet ist und wobei die beiden Randabschnitte in einem gemeinsamen rundbodenförmigen Randabschnitt zum Ummanteln und/oder Umgreifen der Herzspitze zusammenlaufen. Jeder sich in Längsrichtung erstreckende aufgerollte und/oder gefaltete Randabschnitt kann mit Bezug auf den Einführzustand einen geradlinigen Befestigungsabschnitt zur Befestigung mit dem Basisteil und einen an den geradlinigen Befestigungsabschnitt anschließenden aus der Ebene des Basisteils herausgebogenen und nicht an dem Basisteil befestigten freien Abschnitt aufweisen, wobei die auf beiden Längsseiten des Basisteils angeordneten freien Abschnitte in dem rundbodenförmigen Randabschnitt zusammenlaufen. Darüber hinaus sind die freien Abschnitte mit Bezug auf den Einführzustand vorzugsweise in der Ebene des Basisteils gegenüber den geradlinigen Befestigungsabschnitten nach außen abgewinkelt und/oder abgebogen. Zwischen den geradlinigen Befestigungsabschnitten kann dabei eine Aussparung zur Aufnahme und Befestigung des Basisteils gebildet sein, was die sichere Befestigung des Basisteils an dem Ummantelungsteil einerseits und eine kleine Baugröße im Einführzustand sowie die erforderliche Formanpassung des Ummantelungsteils andererseits zuläßt.

Vorzugsweise ist es so, daß die Stabilisierungsschicht die beiden Herzkammern des Herzens im Funktionszustand im wesentlichen vollflächig ummantelt bzw. umgreift. Das erfindungsgemäße System weist neben der Vorrichtung zumindest eine Hilfseinrichtung auf, um die minimal invasive Einbringung der erfindungsgemäßen Vorrichtung in den Körper zu ermöglichen.

Der Erfindung liegt der Grundgedanke zugrunde, eine Vorrichtung zur Unterstützung und/oder zur Übernahme der Pumpfunktion des Herzens zur Verfügung zu stellen, die innerhalb der Brusthöhle entfaltbar ist. Dadurch läßt sich die erfindungsgemäße Vorrichtung chirurgisch wenig invasiv und wenig anspruchsvoll applizieren und ermöglicht eine schnell verfügbare adäquate Unterstützung der Pumpfunktion des Herzens, insbesondere in Akutsituationen, beispielsweise bei akuter Herzinsuffizienz mit einem Cardiac-output von kleiner 50 % bis zum vollständigen Verlust der mechanischen Herzfunktion. Der Ummantelungsteil läßt sich bei der erfindungsgemäßen Vorrichtung durch die inflatierbare und deflatierbare Stabilisierungsschicht aufrollen bzw. auffalten, wobei das Herz und/oder das Herz im Herzbeutel (Perikard) von dem Ummantelungsteil im Funktionszustand ummantelt, umgriffen bzw. umfaßt wird. Die Stabilisierungsschicht dient in diesem Zusammenhang zum einen zur Sicherstellung einer ausreichenden Steifigkeit bzw. Stabilität der am bzw. um das Herz implantierten Vorrichtung sowie zur Formanpassung an die Herzform. Da das Ummantelungsteil flexibel zusammenfaltbar bzw. zusammenrollbar ist, wird eine minimal invasive Einbringung der Vorrichtung in die Brusthöhle und spätere Entfernung ohne konventionell chirurgische Eröffnung des Brustkorbes ermöglicht. Die Stabilisierungsschicht ist dabei zum Auseinanderfalten bzw. Auseinanderrollen des Ummantelungsteils vorgesehen und entsprechend ausgebildet.

Die Augmentationsschicht weist eine deutlich höhere Dehnbarkeit auf als die Stabilisierungsschicht. Wird zyklisch ein Gas oder Gasgemisch in den Hohlraum zwischen der Stabilisierungsschicht und der Augmentationsschicht eingeleitet, wird bei entsprechend vorhandenem Überdruck im Hohlraum eine nach innen gerichtete Aufwölbung des Hohlraums bewirkt, welche zur Kompression der benachbarten Herzkammern sowie zur Herzdruckmassage und Unterstützung der Herzfunktion dient. Es versteht sich, daß alternativ auch die Augmentationsschicht als solche inflatierbar sein kann.

Vorzugsweise weist der Ummantelungsteil im Funktionszustand eine an die Form des Herzens angepaßte kelchartige Tulpenform auf, wobei das Herz im Funktionszustand von dem Ummantelungsteil von vorne nach hinten über die Seitenwände und, vorzugsweise, über die Herzspitze bis in den Bereich der Hinterwände des Herzens ummantelt und/oder umgriffen wird. Es versteht sich, daß die Stabilisierungsschicht dementsprechend ausgebildet sein muß, um durch Inflatieren bzw. Ausrollen und/oder Entfalten die Formanpassung des Ummantelungsteils beim Überführen von dem Einführzustand in den Funktionszustand zu gewährleisten. Die Augmentationsschicht kann dabei mit Bezug auf den Funktionszustand lediglich im Bereich oberhalb der Seitenwände des Herzens und der Hinterwände der Herzkammern vorgesehen sein, so daß der Hohlraum für die zyklische Kompression und Dekompression des Herzens im Bereich der Seitenwände und der Hinterwände des Herzens gebildet wird. Bei einer anderen Ausführungsform der Erfindung kann auch vorgesehen sein, daß die erfindungsgemäße Vorrichtung den Bereich der Herzspitze freiläßt bzw. nicht ummantelt bzw. nicht umgreift.

Die zur Unterstützung und/oder Übernahme der Pumpfunktion des Herzens erforderliche Steifigkeit des Ummantelungsteils wird durch Einleiten eines Gases oder einer geeigneten Flüssigkeit in inflatierbare Bereiche der Stabilisierungsschicht erzeugt. In diesem Zusammenhang kann der Ummantelungsteil im Einführzustand einen von außen nach innen spiralförmig aufgerollten und/oder gefalteten inflatierbaren Randabschnitt aufweisen. Durch Einleiten eines Gases oder einer geeigneten Flüssigkeit in die Stabilisicrungssehicht läßt sich der Randbereich des Ummantelungsteils entsprechend abrollen bzw. entfalten, um die Formanpassung des Ummantelungsteils an die Form des Herzens zu erreichen. Der Ummantelungsteil kann vorzugsweise ausgehend von beiden Längsrändern in Richtung zur Mitte des Ummantelungsteils aufgerollt bzw. eingefaltet sein, so daß sich der Ummantelungsteil auf eine sehr kleine Einführgröße im Einführzustand zusammenrollen bzw. zusammenfalten läßt, was die minimal invasive Einbringung in die Brusthöhle und die spätere Entfemung aus der Brusthöhle ohne konventionell chirurgische Öffnung des Brustkorbes vereinfacht.

Um bei der Unterstützung und/oder Übernahme der Pumpfunktion des Herzens die großen Herzgefäße nicht durch die erfindungsgemäße Vorrichtung zu beschädigen bzw. zu verletzen, ist vorzugsweise vorgesehen, daß der Ummantelungsteil im Funktionszustand auf der Hinterseite, d.h. auf der den Hinterwänden des Herzens zugewandten Seite, eine nach oben geöffnete vorzugsweise hyperbelförmige Aussparung für die großen Herzgefäße aufweist.

Darüber hinaus weist die erfindungsgemäße Vorrichtung Mittel auf, die eine reversible Befestigung bzw. Fixierung der implantierten Vorrichtung in der Brusthöhle ermöglichen, ohne daß die Vorrichtung am Herzen selbst oder an dem Herzbeutel, den Lungen oder den großen Gefäßen in der Brusthöhe befestigt wird. Hierzu ist wenigstens ein Befestigungsmittel zur herz- und/oder herzbeutelfreien Befestigung des Ummantelungsteils vorgesehen, vorzugsweise zur Befestigung an einer inneren Wand des Brustkorbes und/oder an wenigstens einer Rippe des Patienten. Als Befestigungsmittel kann vorzugsweise eine Mehrzahl von Saugnäpfen zum Ansaugen an der inneren Wand des Brustkorbes vorgesehen sein, so daß die Vorrichtung an der inneren Wand des Brustkorbes lagegetreu über dem Herzen befestigt werden kann. Durch die herz- und herzbeutelfreie Befestigung des Ummantelungsteils bzw. der erfindungsgemäßen Vorrichtung wird die Beweglichkeit des schlagenden Herzens trotz der Unterstützung der Pumpfunktion nicht behindert.

Die Befestigung kann alternativ auch am Zwischenrippenraum erfolgen durch Klammerung oder Spreizung an den umliegenden Rippen abgestützt oder durch zwei gegenseitige verschraubte Platten innerhalb und außerhalb des Brustkorbes. Auch ist eine Befestigung am Brustbein und/oder am Rippenbogen durch Klammerungen, Festklemmen, Verschraubungen oder Festnähen möglich. Darüber hinaus kann eine perkutane Befestigung vorgesehen sein, wobei die erfindungsgemäße Vorrichtung ein distales Ende aufweisen kann, welches in der Haut befestigt wird, beispielsweise durch Vernähen, Kleben oder Klammern. Die Befestigung kann schließlich durch an der Vorrichtung befindliche Mikrohäkchen oder Adhäsionselemente oder durch reversibles Verkleben an der inneren Brustwand erfolgen. Weiterhin ist eine Befestigung am Zwerchfell möglich, wobei sich die erfindungsgemäße Vorrichtung auf dieser Muskelplatte mit ihrem distalen Ende abstützen und durch Vernähen, Kammern oder Festhaken befestigt werden kann.

Ist als Befestigungsmittel eine Mehrzahl von Saugnäpfen vorgesehen, sind diese mit wenigstens einer Fluidleitung zum Absaugen bzw. Evakuieren der zwischen den Saugnäpfen und einer inneren Wand des Brustkorbes gebildeten Ansaugräume verbunden. Durch Absaugen ist es möglich, den Druck in den Ansaugräumen zu verringern und damit die erfindungsgemäße Vorrichtung lagegetreu über dem Herzen an der inneren Wand des Brustkorbes zu befestigen. Darüber hinaus kann über die Saugnäpfe auch eine Gleitflüssigkeit zugeführt werden, um bessere Gleiteigenschaften zu erzielen.

Bei einer besonders bevorzugten Ausführungsform der erfindungsgemäßen Vorrichtung ist auf der Vorderseite des Ummantelungsteils im Bereich der Mittellängsachse ein mit dem Ummantelungsteil verbundener nicht inflatierbarer Basisteil vorgesehen, wobei der Basisteil das Befestigungsmittel aufweist und wobei, vorzugsweise, sich der Basisteil mit Bezug auf die Anordnung im Funktionszustand parallel zur Herzachse von einem oberen Rand des Ummantelungsteils über die Herzbasis nach unten in Richtung zur Herzspitze erstreckt. Der Basisteil ist zur Befestigung der Vorrichtung in der Brusthöhle bzw. zur Halterung des Ummantelungsteils vorgesehen. Basisteil und Ummantelungsteil sind vorzugsweise unterschiedliche Bauteile mit unterschiedlichem Schichtaufbau und/oder unterschiedlicher Steifigkeit, die fest miteinander verbunden sind. Vorzugsweise weist der Basisteil als Halteelement eine Mehrzahl von Saugnäpfen auf, die flächig über den Basisteil verteilt angeordnet sind. Am Basisteil können darüber hinaus Mikrohäkchen oder Adhäsionselemente vorgesehen sein, um die erfindungsgemäße Vorrichtung an der inneren Brustwand zu befestigen. Zwischen dem Ummantelungsteil und dem Basisteil kann eine vollständige, auf der gesamten Länge des Basisteils bestehende Verbindung vorgesehen sein. Diese Verbindung zwischen den beiden Teilen dient in erster Linie der Fixierung des Ummantelungsteils über den Basisteil an der inneren Wand des Brustkorbes. Per Basisteil kann dabei über seine gesamte Länge auf dem Ummantelungsteil aufliegen, wobei die Verbindung zwischen beiden Teilen auf der gesamten Unterfläche oder zumindest einem Teil der Unterfläche des Basisteils vorgesehen ist. Als Unterfläche ist hier die dem Herzen zugewandte Seite des Basisteils zu verstehen. Grundsätzlich ist es natürlich auch möglich, daß der Basisteil mit dem Ummantelungsteil nur teilweise, d.h. abschnittsweise über seine Länge, verbunden ist.

Im übrigen kann der Basisteil wenigstens eine äußere zur Applikation einer Gleitflüssigkeit auf die Innen- und/oder Außenseite des Basisteils ausgebildete Applikationsschicht mit einem inneren Kanalsystem und porenartigen Öffnungen zur Innenseite und/oder zur Außenseite aufweisen. Es versteht sich, daß das innere Kanalsystem mit wenigstens einer Fluidleitung entsprechend verbunden ist, um eine Gleitflüssigkeit über die Applikationsschicht auszutragen. Durch das Austragen einer Gleitflüssigkeit können auftretende Reibungskräfte verringert und eine Gewebeschädigung verhindert werden. Liegt der Basisteil auf seiner Innenseite unmittelbar gegen die Herzoberfläche an, so kann durch Austrag einer Gleitflüssigkeit auf der Innenseite das Aneinandergleiten der Herzoberfläche und der Innenoberfläche der erfindungsgemäßen Vorrichtung bei minimal auftretenden Reibungskräften ermöglicht werden.

Der Basisteil kann eine langgestreckte Form aufweisen, wobei die Länge des Basisteils vorzugsweise ca. 2/3 der Länge der Vorderseite des Ummantelungsteils mit Bezug auf den Funktionszustand entspricht. Dies vereinfacht die Befestigung des Basisteils an der inneren Brustwand.

Im übrigen ist der Basisteil vorzugsweise derart elastisch ausgebildet, daß sich der Basisteil beim Befestigen an der inneren Wand des Brustkorbes an die Wölbung der inneren Wand anpaßt. Dadurch wird eine sichere Befestigung des Basisteils vereinfacht, wobei sich die beispielsweise zur Befestigung des Basisteils an der inneren Wand vorgesehenen Saugnäpfe vollflächig an der inneren Wand festsaugen können.

Um die für die Herzunterstützung erforderliche Formanpassung des Ummantelungsteils beim Überführen von dem Einführzustand in den Funktionszustand einerseits und eine möglichst kleine Baugröße der erfindungsgemäßen Vorrichtung im Einführzustand andererseits sicherzustellen, ist bei einer bevorzugten Ausführungsform der Basisteil mit Bezug auf die Anordnung im Funktionszustand lediglich im Bereich zwischen dem oberen Rand des Ummantelungsteils und der Herzbasis mit dem Ummantelungsteil verbunden.

Dadurch wird die Formanpassung des Ummantelungsteils nicht durch die Befestigung mit dem Basisteil behindert, gleichzeitig aber eine sichere Halterung des Ummantelungsteils sichergestellt.

Die Stabilisierungsschicht kann eine Wabenstruktur mit einer Mehrzahl von nebeneinanderliegenden Wabenkammern aufweisen, wobei die Wabenkammern mit wenigstens einer Fluidleitung zur zyklischen Zufuhr eines Fluides in die Wabenkammern verbunden sind, wobei, vorzugsweise, die Wabenkammern einen konisch in Richtung zum Herzen zulaufenden Querschnitt mit größerer Grundfläche auf der herzfernen Seite und mit kleinerer Grundfläche auf der herznahen Seite aufweisen und wobei bei Zufuhr eines Fluides in die Wabenkammern die Wabenkammern inflatieren und eine nach innen gerichtete Wölbung des Ummantelungsteils bewirkt wird. Die Stabilisierungsschicht ist wabenartig strukturiert und wenig oder gar nicht dehnbar. Beim Einfüllen eines komprimierten Gases oder Gasgemisches in die Wabenstruktur wird ein erhöhter Gasdruck innerhalb der Wabenkammern erzeugt. Dieser erhöhte Gasdruck verleiht der im Brustkorb implantierten erfindungsgemäßen Vorrichtung die notwendige Form (Krümmung), um das Herz zu ummanteln bzw. zu umgreifen bzw. zu umspannen. Gleichzeitig wird der erfindungsgemäßen Vorrichtung durch den Überdruck in den Wabenkammern die notwenige Steifigkeit verliehen, die erforderlich ist, um die Pumpfunktion des Herzens zu unterstützen oder zu übernehmen.

Vorzugsweise weist der Ummantelungsteil eine Stabilisierungsschicht mit einer entsprechenden Wabenstruktur auf, was zu einem einfachen konstruktiven Aufbau der erfindungsgemäßen Vorrichtung und zu geringen Herstellungskosten führt. Grundsätzlich können jedoch auch mehrer Stabilisierungsschichten mit wabenartigen Kammern vorgesehen sein, wobei sich die Schichten vollständig überlagern oder nur teilweise überlappen und einen einem Dachziegelverbund ähnlichen Aufbau aufweisen können. Die Stabilisierungsschicht erstreckt sich vorzugsweise über die gesamte Fläche des Ummantelungsteils. Grundsätzlich ist aber auch möglich, daß sich die Stabilisierungsschicht nur bereichsweise über die Fläche des Ummantelungsteils erstreckt. In diesem Zusammenhang kann das Ummantelungsteil das Herz im Funktionszustand auch nur fingerartig umgreifen bzw. umklammern.

Bei einer weiteren Ausführungsform der Erfindung kann wenigsten ein Stabilisierungsdraht für den Ummantelungsteil vorgesehen sein, wobei, vorzugsweise, der Stabilisierungsdraht zwischen der Stabilisierungsschicht und der Augmentationsschicht angeordnet ist. Insbesondere kann ein vom Baisteil der Vorrichtung ausgehender und am hinteren Pol der Umgreifung des Ummantelungsteils um die Herzspitze ansetzender Stabilisierungsdraht vorgesehen sein, der als zusätzliche Stabilisierungsstütze für den Ummantelungsteil sowie als Unterstützungselement bei der Einbringung der erfindungsgemäßen Vorrichtung in den Körper vorgesehen sein kann. Mit dem Stabilisierungsdraht kann der untere Bereich des Ummantelungsteils während der Einbringung der Vorrichtung um die Herzspitze gezogen werden. Dadurch wird das minimal invasive Einbringen der erfindungsgemäßen Vorrichtung in den Körper vereinfacht.

Zusätzlich zur Stabilisierungsschicht kann wenigstens eine mit der Stabilisierungsschicht verbundene Versorgungsschicht zur Zufuhr eines Fluides in die Wabenkammern vorgesehen sein. Vorzugsweise erfolgt die Versorgung der Wabenkammern mit den Fluid über drei voreinander getrennte Versorgungs- bzw. Fluidleitungen, wobei drei benachbarte Wabenkammern durch unterschiedliche Versorgungsleistungen mit dem Fluid befüllbar sind. Dadurch wird sichergestellt, daß es auch bei einer Leckage in einer Wabenkammer nicht zu einem Druckverlust in einer benachbarten Wabenkammer kommen kann, so daß die Steifigkeit und Stabilität des Ummantelungsteils auch bei einer Leckage aufrecht zu erhalten ist.

Ebenso wie der Basisteil kann der Ummantelungsteil wenigstens eine zur Applikation einer Grleitflüssigkeit auf die Innen- und/oder Außenseite des Ummantelungsteils ausgebildete Applikationsschicht mit einem inneren Kanalsystem und porenartigen Öffnungen zur Innenseite und/oder Außenseite des Funktionsteils aufweisen. Durch die Gleitflüssigkeit werden Reibungskräfte zwischen der der Herzoberfläche zugewandten Innenfläche des Ummantelungsteils und der Herzoberfläche bei der zyklischen Kompression des Herzens verringert. In diesem Zusammenhang soll vorzugsweise ein Verschiebespalt zwischen der Herzoberfläche und der Innenfläche der erfindungsgemäßen Vorrichtung geschaffen werden, wobei in diesem Verschiebespalt bzw. Raum von extrakorporalen Flüssigkeiten, Gele und/oder Medikamente und/oder andere Substanzen appliziert werden können. Vorzugsweise ist die Applikation einer Gleitflüssigkeit auf der Innenseite und auf der Außenseite des Ummantelungsteils einerseits und des Basisteils andererseits vorgesehen, so daß ein direkter Kontaktschluß zwischen der Innenfläche der erfindungsgemäßen Vorrichtung und dem Herzen bzw. dem Herzen im Herzbeutel sowie der Außenfläche der erfindungsgemäßen Vorrichtung und den angrenzenden Lungen vermieden wird. Durch porenartige Öffnungen an der Innenfläche und/oder Außenfläche der Vorrichtung können kontinuierlich oder temporär Flüssigkeiten oder Gele abgegeben werden, um Reibungskräfte zu verringern. Je nach Stärke des applizierten Flüssigkeits- oder Gelfilms kann ein direkter Kontaktschluß zwischen der Innenfläche der erfindungsgemäßen Vorrichtung mit dem Herzen sowie der Außenfläche der Vorrichtung mit dem angrenzenden Lungengewebe vollständig verhindert werden.

Die Applikationsschicht erstreckt sich vorzugsweise über die gesamte Fläche des Ummantelungsteils. Im Ergebnis wird der Ummantelungsteil bei der erfindungsgemäßen Vorrichtung durch eine Stabilisierungsschicht gebildet, die vorzugsweise beidseitig mit einer Applikationsschicht ausgestattet ist, wobei im Bereich oberhalb der Seitenwände des Herzens und der Hinterwände der Herzkammern (mit Bezug auf den Funktionszustand des Ummantelungsteils) zwischen der Stabilisierungsschicht und der Applikationsschicht die Augmentationsschicht vorgesehen ist, um in diesem Bereich den Hohlraum für die zyklische Kompression des Herzens ausbilden zu können.

An der inneren Oberfläche des Ummantelungsteils und/oder des Basisteils können ein oder mehrere Elektroden angebracht sein, um eine Schrittmacherfunktion in die Vorrichtung zu integrieren bzw. bei mindestens zwei Elektroden direkt Potentiale für die Darstellung eines EKGs abzuleisten. Werden mindestens drei Elektroden in die Vorrichtung integriert und räumlich angeordnet, lassen sich damit dreidimensionale EKG-Ableitungen vornehmen.

Die minimal invasive Einbringung und Entfernung der erfindungsgemäßen Vorrichtung wird nachfolgend erläutert.

### 1. Bildgebung

Durch bildgebende Verfahren während des gesamten Einführungsvorganges, wie bspw. eine röntgenologische Darstellung des Brustkorbes, erhält der behandelnde Mediziner vor der Einbringung der Vorrichtung Aufschluß über die Formen, die Lagebeziehungen und Größe der Organe in der Brusthöhle sowie während der Einführung der erfindungsgemäßen Vorrichtung über die Position der eingebrachten Hilfsmittel und der Vorrichtung im Körperinneren am Herzen.

### 2. Auswahl der verwendeten Ausführungsvariante der Vorrichtung nach Form und Größe

Durch Inspektion der vorliegenden Brustkorbdarstellung trifft der behandelnde Mediziner anhand der gewonnenen Erkenntnisse die Auswahl, welche Ausführungsform sowie -größe beim vorliegenden Patienten zum Einsatz gelangen kann. Er kann dabei im Hinblick auf die festgestellte Herzform vorzugsweise auf 4 Grundformen der Vorrichtung zurückgreifen, welche eine Auswahl an mehrere Untervarianten mit unterschiedlichen Größenausführungen (klein, mittel, groß, sehr groß) haben können. Die Grundformen sind

| | |
|---|---|
| Normaltyp | - keine auffällige Formveränderungen von der Normalform des Herzens (kegelförmiger Zylinder) erkennbar; |
| Linkstyp | - auffällige Vergrößerung der linken Herzkammer (bspw. bei langjährig bestehendem chronischen Bluthochdruck); |
| Rechtstyp | - auffällige Vergrößerung der rechten Herzkammer |

| | |
|---|---|
| | (bspw. bei langjährig bestehenden chronischen Lungenerkrankungen); |
| Rundtyp | - Vergrößerung des gesamten Herzens bzw. stark detailliertes Herz. |

### 3. transkutaner Einführungskanal durch die Haut des Oberbauchs

Nach Desinfektion der Haut des Oberbauches wird durch einen kleinen Schnitt (3 - 5 cm) ein Zugang im Bereich unter dem Schwertfortsatz des Brustbeins (Processus xiphoideus) zur Bauchhöhle geschaffen.

### 4. Sondierung des Herzens

Durch den geschaffenen Zugang zum Oberbauch kann eine spatelartige Sondierungseinrichtung, die Bestandteil des erfindungsgemäßen Systems ist, in Richtung der Brusthöhle bis zum Herz vorgeschoben werden. Dabei wird das Zwerchfell (Diaphragma) in der Regel durch einen anatomisch vorhandenen Spalt (Lareyischer Spalt) passiert, um in die Brusthöhle zu gelangen. Der Lareyische Spalt befindet sich im vorderen Areal des Zwerchfells. Die spatelartige Sondierungseinrichtung wird im Regelfall nach dem Passieren des Zwerchfells nach rechts geschwenkt (aus Sicht des einführenden Mediziners nach rechts in die linke Brusthöhlenseite unter Berücksichtigung der anatomischen Seiten), um zur Herzspitze zu gelangen.

### 5. Umfahren der Vorderwand des Herzens

Bei Erreichen und Sondierung des Herzens wird die spatelartige Sondierungseinrichtung vorsichtig zwischen Vorderwand des Herzens und innere Brustwand geschoben. Eventuelle Verwachsungen des Herzbeutels und der inneren Brustwand können mit der spatelartigen Sondierungseinrichtung stumpf abgelöst werden. Nach Umfahren der Vorderwand des Herzens und stumpfer Ablösung eventueller Verwachsungen wird die Sondierungseinrichtung aus dem Körper durch Zurückziehen entfernt.

### 6. Einbringung der erfindungsgemäßen Vorrichtung

Eine rohrartige Einführeinrichtung, die ebenfalls Bestandteil des erfindungsgemäßen Systems ist, wird durch den Zugang im Oberbauch mit dem proximalen Ende eingebracht und bis zur Herzspitze vorgeschoben. Dann wird eine bewegliche Positionierungseinrichtung, die einen weiteren Bestandteil des erfindungsgemäßen Systems bildet, mit der am proximalen Ende befestigten erfindungsgemäßen Vorrichtung durch einen Einführkanal der rohrartigen Einführeinrichtung bis zum Erreichen der Herzspitze vorgeschoben. Jetzt wird die Einführeinrichtung einige Zentimeter zurückgezogen, so daß ein bewegliches Gelenk am proximalen Ende der Positionierungseinrichtung in der Brusthöhle aus dem proximalen Ende der Einführeinrichtung austritt. Durch weiteren Vorschub und durch die Beweglichkeit des Gelenkes am proximalen Ende der Positionierungseinrichtung kann jetzt die erfindungsgemäße Vorrichtung vor dem Herzen in Richtung der Herzachse positioniert werden. Die Fluid-Zuleitungen und Ableitungen der erfindungsgemäßen Vorrichtung werden an einer extrakorporal angeordneten Fluidversorgungs- und Steuerungseinrichtung angeschlossen. Ist dies erfolgt; wird der Ummantelungsteil mittels Gasdruck um das Herz entfaltet und die Vorrichtung mittels des Sogs im Basisteil der Vorrichtung an der inneren Brustwand fixiert. Die bewegliche Positionierungseinrichtung wird durch den Einführkanal der Einführeinrichtung hindurch aus dem Körper entfernt. Die rohrartige Einführeinrichtung wird über die Zu- und Ableitungen aus dem Körper herausgezogen und kann außerhalb des Körpers in zwei Halbschalen getrennt und somit von den Zu- und Ableitungen der erfindungsgemäßen Vorrichtung abgenommen werden.

### 7. Applikation von Flüssigkeiten, Flüssigkeitsgemischen oder Gelen

Nach erfolgter Einbringung, Positionierung und reversiblen Befestigung der erfindungsgemäßen Vorrichtung an der inneren Brustwand wird eine Flüssigkeit oder ein Flüssigkeitsgemisch oder ein Gel auf die innere und äußere Oberfläche der Vorrichtung über die jeweilige Applikationsschicht des Ummantelungsteils appliziert. Durch den benetzenden Flüssigkeitsfilm soll zwischen der inneren Oberfläche der Vorrichtung und dem Herzen sowie der äußeren Oberfläche und den angrenzenden Lungen jeweils ein Verschiebespalt geschaffen werden, um Reibungskräfte weitestgehend zu reduzieren.

### 8. zyklisches Pumpen der Augmentationsschicht

Durch zyklisches Pumpen mit einem Gas- oder Gasgemisch in die Augmentationsschicht des Ummantelungsteils kann eine direkte Unterstützung der Pumpfunktion des Herzens erfolgen.

Die minimal invasive Entfernung der erfindungsgemäßen Vorrichtung aus dem Körper sieht folgende Verfahrensschritte vor:

### 1. Evakuierung von Gasen und Gasgemischen

Aus sämtlichen Schichten des Ummantelungsteils werden die Gase und/oder Gasgemische evakuiert, was zum Deflatieren der Stabilisierungsschicht führt.

### 2. Lösen der Fixierung an der inneren Brustwand

Der Sog im Bereich des Basisteils zur Befestigung der Vorrichtung an der inneren Brustwand wird aufgehoben.

### 3. Herausziehen der zusammenfaltbaren Vorrichtung

Da der Ummantelteil aus zusammenfaltbaren und zusammenrollbaren Materialien gestaltet ist, läßt sich die erfindungsgemäße Vorrichtung nach Schritt 1 und 2 des Entfernungsvorganges, ähnlich einer Drainagenentfernung aus einer Körperhöhle, durch Ziehen an den Zu- bzw. Ableitungen aus dem Körper entfernen.

### 4. Hautnaht

Die Durchtrittstelle in das Körperinnere am Oberbauch wird nach Entfernung der Vorrichtung vernäht.

Nachfolgend werden Ausführungsbeispiele der Erfindung anhand der Zeichnungen näher beschrieben. Dabei zeigt
- Fig. 1: eine perspektivische Darstellung einer erfindungs gemäßen Vorrichtung zur Unterstützung und/oder Übernahme der Pumpfunktion des Herzens im Funktionszustand eines Ummantelungsteils der Vorrichtung;
- Fig. 2: die in Fig. 1 dargestellte Vorrichtung im Funktionszustand bei zyklischer Kompression und Dekompression des Herzens in einer Ansicht von vorne,
- Fig. 3: die in Fig. 1 dargestellte Vorrichtung im Funktionszustand bei der zyklischen Kompression und Dekompression des Herzens in einer Ansicht von der Seite,
- Fig. 4: die in Fig. 1 dargestellte Vorrichtung im zusammengefalteten Einführzustand in einer Ansicht von oben,
- Fig. 5: die in Fig. 4 dargestellte Vorrichtung in einer Querschnittsansicht entlang der Schnittlinie I-I,
- Fig. 6: die in Fig. 4 dargestellte Vorrichtung in einer Seitenansicht,
- Fig. 7: die in Fig. 1 dargestellte Vorrichtung in einer Querschnittsansicht im Funktionszustand des Ummantelungsteils,
- Fig. 8: eine schematische Darstellung des Schichtaufbaus des Ummantelungsteils der in Fig. 1 dargestellten Vorrichtung im deflatierten Einführzustand des Ummantelungsteils,

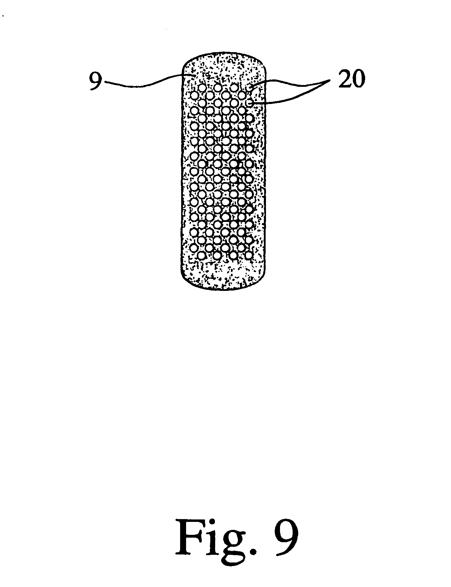

- Fig. 9: eine Draufsicht auf ein Basisteil der in Fig. 1 dargestellten Vorrichtung,

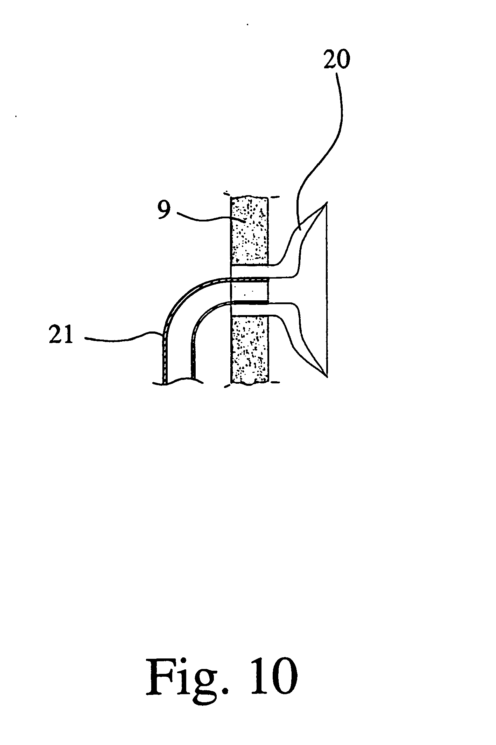

- Fig. 10: eine schematische Darstellung eines Saugnapfes an dem in Fig. 9 dargestellten Basisteil zur Befestigung des Basisteils an einer Innenwand des Brustkorbes,
- Fig. 11: das in Fig. 9 dargestellte Basisteil in einer schematischen Darstellung im an eine Innenwand des Brustkorbes angesaugten Zustand,
- Fig. 12: eine schematische Querschnittsansicht einer Stabilisierungsschicht des Ummantelungsteils der in Fig. 1 dargestellten Vorrichtung im Funktionszustand des Ummantelungsteils,
- Fig. 13: eine schematische Darstellung der in Fig. 12 dargestellten Stabilisierungsschicht mit wabenartiger Struktur,
- Fig. 14a,: b eine Draufsicht und eine perspektivische Ansicht einer Applikationsschicht der in Fig. 1 dargestellten Vorrichtung zur Applikation einer Gleitflüssigkeit,
- Fig. 15: eine schematische Querschnittsansicht einer Positionierungseinrichtung zur Positionierung der in Fig. 1 dargestellten Vorrichtung,
- Fig. 16a, b: ein Kopfgelenk der in Fig. 15 dargestellten Positionierungseinrichtung im an den in Fig. 9 dargestellten Basisteil angekoppelten und im entkoppelten Zustand,
- Fig. 17: eine spatelartige Sondierungseinrichtung,
- Fig. 18a,b,c: eine rohrförmige mehrteilige Einführeinrichtung zum Einführen der in Fig. 1 dargestellten Vorrichtung in die Brusthöhle im Einfuhrzustand des Ummantelungsteils in einer Querschnittsansicht, einer Längsschnittansicht und einer Draufsicht, wobei in Fig. 18c die Einführeinrichtung im geteilten Zustand dargestellt ist,
- Fig. 19: eine schematische Ansicht der in Fig. 1 dargestellten Vorrichtung mit einem Stabilisierungsdraht für den Ummantelungsteil und
- Fig. 20: eine schematische Darstellung der Fixierung des in Fig. 9 dargestellten Basisteils an den Rippen des Brustkorbes.

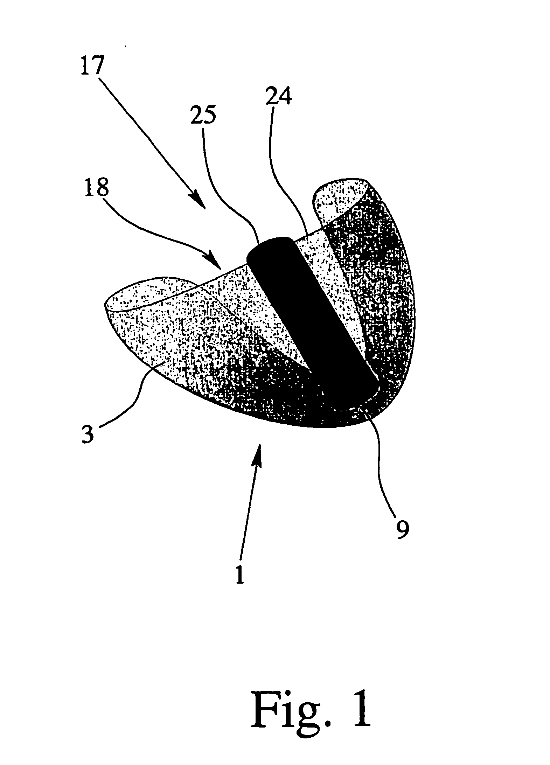

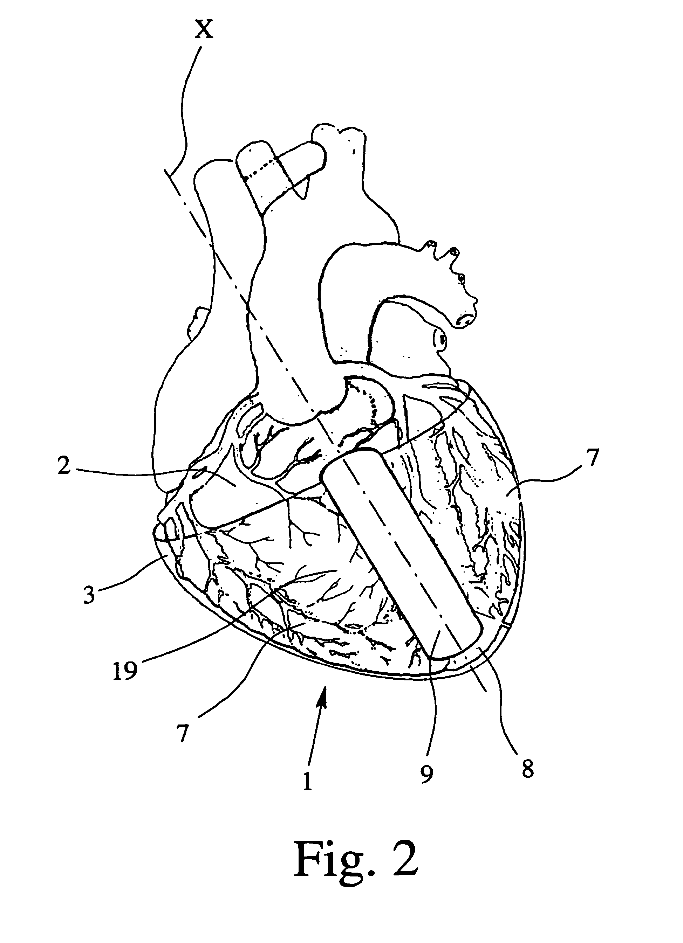

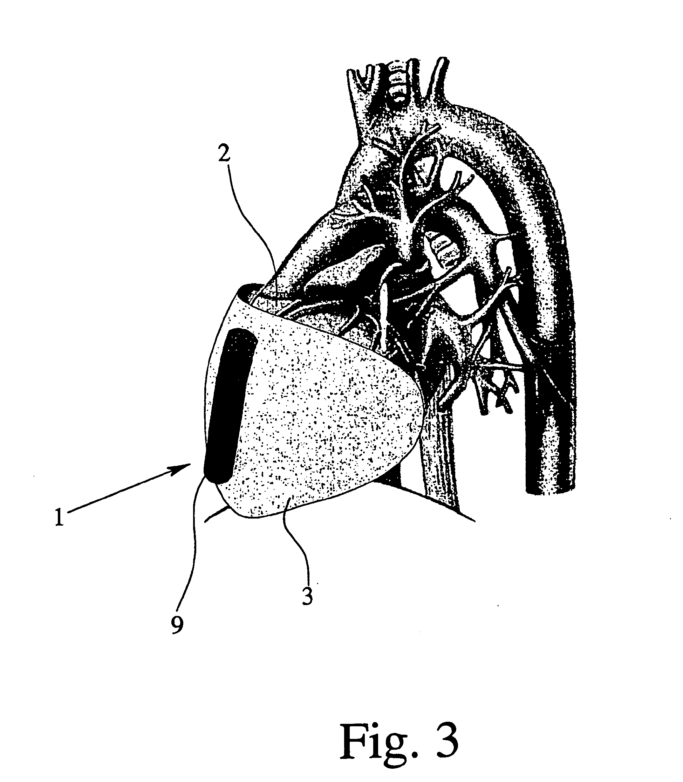

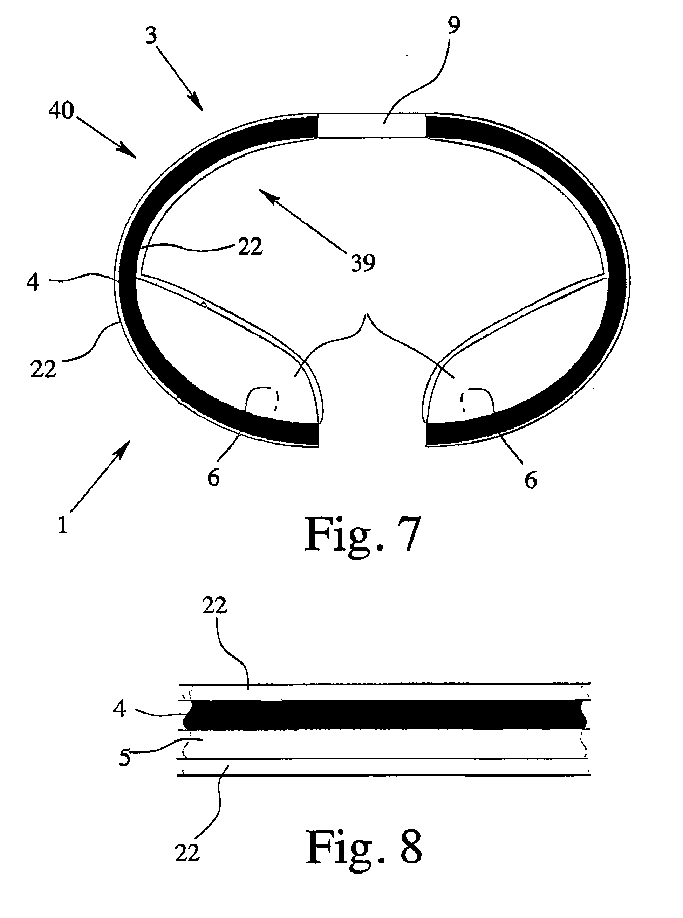

In Fig. 1 ist eine Vorrichtung 1 zur Unterstützung und/oder Übernahme der Pumpfunktion eines in den Fig. 2 und 3 gezeigten Herzens 2 mit einem mehrschichtigen Ummantelungsteil 3 dargestellt, wobei der Ummantelungsteil 3 zur Kompression des Herzens ausgebildet ist und wobei der Ummantelungsteil 3 eine in den Fig. 7, 8 und 12 dargestellte Stabilisierungsschicht 4 zur Formgebung des Ummantelungsteils 3 aufweist. Darüber hinaus weist der Ummantelungsteil 3 eine innere dehnbare Augmentationsschicht 5 auf, die in den Fig. 7 und 8 dargestellt ist. Zwischen der Stabilisierungsschicht 4 und der Augmentationsschicht 5 wird wenigstens ein mittels eines Fluides inflatierbarer und anschließend deflatierbarer Hohlraum 6 für die zyklische Kompression des Herzens 2 gebildet, wobei der Hohlraum 6 im inflatierten Zustand in Fig. 7 dargestellt ist.

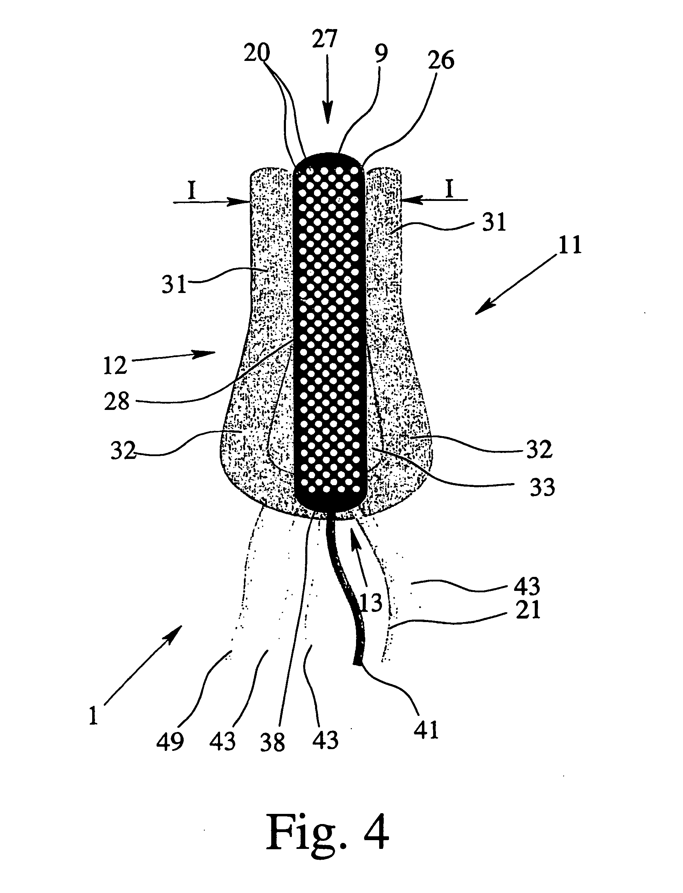

Die Stabilisierungsschicht 4 ist mittels eines Fluides zumindest bereichsweise inflatierbar, um den Ummantelungsteil 3 von einem zusammengerollten und/oder zusammengefalteten Einführzustand der Vorrichtung 1, der beispielsweise in Fig. 4 dargestellt ist und das Einführen der Vorrichtung 1 in einen Körper ermöglicht, ist einen gewölbten Funktionszustand zu überführen, der in Fig. 1 dargestellt ist. Im Funktionszustand wird das Herz 2 von dem Ummantelungsteil 3 zumindest teilweise ummantelt und/oder umgriffen.

In den Fig. 2 und 3 ist die Vorrichtung 1 nach dem minimal invasiven Einbringen in die Brusthöhle eines Patienten im Zustand der zyklischen Kompression des Herzens 1 von vorne (Fig. 2) und von der Seite (Fig. 3) dargestellt. Wie sich aus den Fig. 2 und 3 ergibt, weist der Ummantelungsteil 3 der Vorrichtung 1 im Funktionszustand eine an die Form des Herzens 2 angepaßte kelchartige Tulpenform auf, so daß das Herz 2 im Funktionszustand von dem Ummantelungsteil 3 von vorne nach hinten über die Seitenwände 7 des Herzens 2 und über die Herzspitze 8 bis in den Bereich der Hinterwände des Herzens 2 ummantelt wird.

Die Vorrichtung 1 weist neben dem Ummantelungsteil 3 einen Basisteil 9 auf. Der Basisteil 9 ist zur Befestigung der Vorrichtung 1 an dem Brustkorb ausgebildet und liegt im Funktionszustand in Richtung der Herzachse X, die in Fig. 2 dargestellt ist, vor dem Herzen 2. Die Ummantelung des Herzens 2 wird dabei weitestgehend vom Ummantelungsteil 3, ausgehend von den Seiten sowie vom unteren Rand des Basisteils 9 ausgehend über die Seiten des Herzens 2 und um die Herzspitze 8 herum vollzogen. Eine in den Fig. 1 bis 3 nicht dargestellte Saugvorrichtung am Basisteil 9 stellt die Befestigung der Vorrichtung 1 an dem Brustkorb sicher. Dies ermöglicht die Fixierung der Vorrichtung 1 ohne Befestigung am Herzen, dem Herzbeutel, den Lungen oder an den großen Gefäßen im Brustkorb.

Der Ummantelungsteil 3 ist mehrschichtig aufgebaut, worauf nachfolgend noch im Zusammenhang mit den Fig. 7 und 8 sowie 12 und 13 näher eingegangen wird. Die Steifigkeit der implantierten und der an dem Brustkorb befestigten Vorrichtung 1 wird durch ein komprimiertes Gas oder Gasgemisch in einer oder mehreren wabenartig strukturierten Schichten des Ummantelungsteils 3 erzeugt. Im Inneren des Ummantelungsteils 3 sind dazu, vorzugsweise im Areal über den Seitenwänden 7 und den Hinterwänden des Herzens 2, ein oder mehrere dehnbare Schichten vorgesehen. Wird in diese dehnbaren Schichten zyklisch ein Gas oder Gasgemisch eingebracht und ein Überdruck erzeugt, kommt es zu einer zyklischen Vorwölbung dieser Schichten und zur zyklischen Kompression der Herzkammern bzw. zur Unterstützung der Herzfunktion.

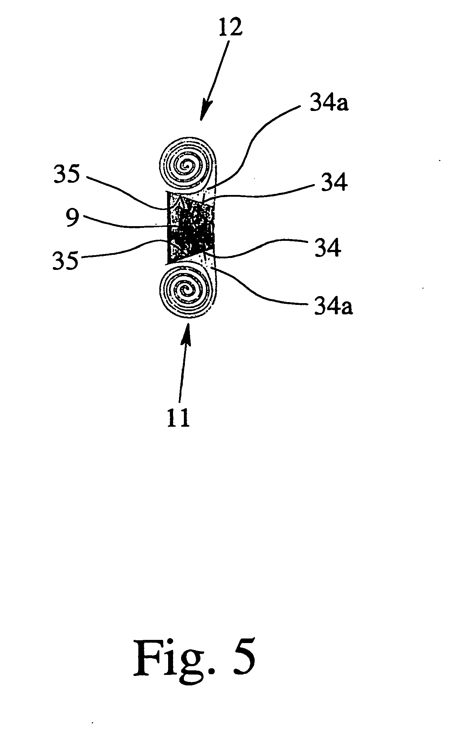

Wie sich insbesondere aus den Fig. 4 und 5 ergibt, weist der Ummantelungsteil 3 mit Bezug auf den Einführzustand zwei sich in Längsrichtung erstreckende von außen nach innen spiralförmig aufgerollte und/oder gegebenenfalls gefaltete Randabschnitte 11, 12 auf, wobei der Basisteil 9 zwischen den Randabschnitten 11, 12 angeordnet ist und wobei die beiden Randabschnitte 11, 12 in einem gemeinsamen mit Bezug auf den Funktionszustand der Vorrichtung 1 der Herzspitze 8 zugewandten unteren rundbodenförmigen Randabschnitt 13 zum Ummanteln und/oder Umgreifen der Herzspitze zusammenlaufen. Die Randabschnitte 11, 12, 13 lassen sich mit einem Fluid in einen inflatierten Zustand überführen, so daß der Ummantelungsteil 3 seine an das Herz 2 angepaßte und in den Fig. 2 und 3 gezeigte Form annimmt.

Der Ummantelungsteil 3 bildet im Funktionszustand auf der Hinterseite 17 der Vorrichtung 1 eine nach oben geöffnete vorzugsweise hyperbelförmige Aussparung 18 aus, damit sich der Ummantelungsteil 3 um das Herz 2 legen kann, ohne daß die großen Herzgefäße auf der Hinterseite 17 des Herzens 2 behindert werden.

Auf der dem Herzen 2 abgewandten und dem Brustkorb zugewandten Seite weist der Basisteil 9 flächig angeordnete Saugnäpfe 20 auf. Die Saugnäpfe 20 stehen über wenigstens eine Zuleitung mit dem extrakorporalen Raum außerhalb des Körpers in Verbindung, wobei über die Zuleitung 21 ein Sog erzeugt werden kann, durch welchen die Vorrichtung 1 an dem Brustkorb angesaugt und fixiert wird. Gleichzeitig kann über die Zuleitung 21 natürlich auch der Sog wieder aufgelöst werden. Die Zuleitung 21 ist vorzugsweise druck- bzw. unterdruckstabil, gas- und flüssigkeitsdicht ausgebildet. Im übrigen ist es über die Zuleitung 21 möglich, unterstützend eine Flüssigkeit einzubringen, wodurch gleichzeitig bessere Gleiteigenschaften gewährleistet werden.

Der Basisteil 9 ist das Halteelement der Vorrichtung 1, um die Vorrichtung 1 unabhängig von einer Befestigung am Herzen 2, an den Lungen oder großen Gefäßen lagegetreu über dem Herzen 2 und vor allem über der Herzbasis 19 zu positionieren. Die Befestigung der Vorrichtung 1 ist somit unabhängig von den vollzogenen Bewegungsexkursionen der Organe der Brusthöhle möglich, vor allem unabhängig von den Herzbewegungen, wobei die Herzspitze 8 sich während der Kontraktion in Richtung der Herzbasis 19 bewegt und dabei eine Drehung von annähernd 23° vollzieht. Der Basisteil 9 ist darüber hinaus formanpassungsfähig, so daß er beim Ansaugen bzw. Fixieren über die Saugnäpfe 20 an den Brustkorb die Wölbung der inneren Thoraxwand annimmt.

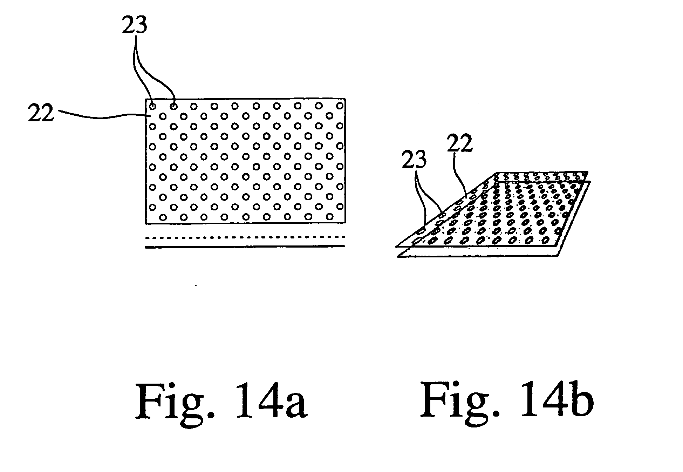

Auf der dem Herzen 2 zugewandten Seite des Basisteils 9 kann eine Applikationsschicht 22 aufgebracht sein, did ein netzartiges Kanälchensystem mit porenartigen Öffnungen 23 aufweist. Über die Applikationsschicht 22 läßt sich eine Gleitflüssigkeit auf der dem Herzen 2 zugewandten Seite des Basisteils 9 abgeben, um die Gleitreibung zwischen dem Basisteil 9 und dem Herzen 2 im Funktionszustand der Vorrichtung 1 zu verringern. Die Applikationsschicht 22 ist in einer Ansicht von oben in Fig. 14a und in einer perspektivischen Ansicht schräg von oben in Fig. 14b dargestellt.

Der Basisteil 9 erstreckt sich im Funktionszustand des Ummantelungsteils 3 in Richtung der Herzachse X von einem oberen Rand 24 des Ummantelungsteils 3 über die Herzbasis 19 nach unten bis in den Bereich der Herzspitze 8. Dies ist in den Fig. 1 bis 3 dargestellt. Wie sich aus Fig. 1 ergibt, steht der obere Rand 25 des Basisteils 9 über den oberen Rand 24 des Ummantelungsteils 3 über, was das Ankoppeln einer Positioniereinrichtung 52 vereinfacht. Hierauf wird später noch eingegangen.

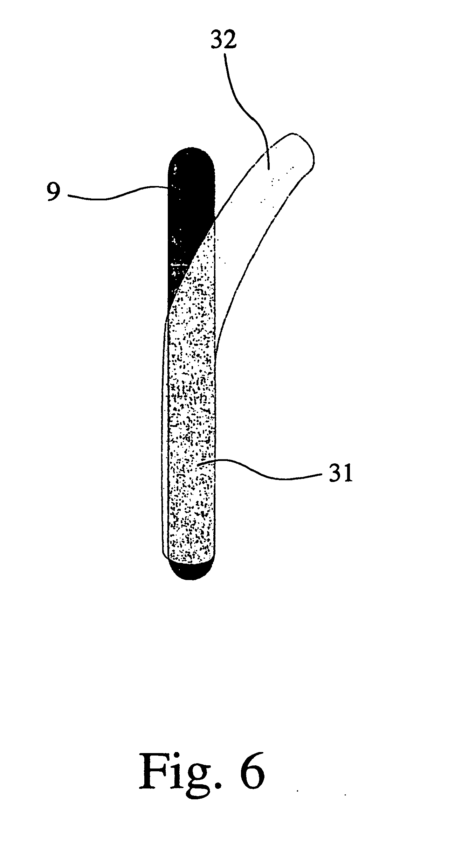

Wie sich insbesondere aus Fig. 3, Fig. 4 und Fig. 6 ergibt, ist der Basisteil 9 bei der dargestellten Ausführungsform lediglich zwischen einem oberen Randbereich 26 auf der von der Herzspitze 8 entfernten Seite 27 des Basisteils 9 und einem im Funktionszustand der Vorrichtung 1 der Herzbasis 19 gegenüberliegenden mittleren Bereich 28 des Basisteils 9 mit dem Ummantelungsteil 3 flächig verbunden. Jeder sich in Längsrichtung erstreckende aufgerollte und/oder gefaltete Randabschnitt 11, 12 des Ummantelungsteils 3 weist mit Bezug auf den in Fig. 4 gezeigten Einführzustand einen geradlinigen Befestigungsabschnitt 31 zur Befestigung mit dem Basisteil 9 und einen an den geradlinigen Befestigungsabschnitt 31 anschließenden aus der Ebene des Basisteils 9 herausgebogenen und nicht an dem Basisteil 9 befestigten freien Abschnitt 32 auf, wobei die auf beiden Längsseiten des Basisteils 9 angeordneten freien Abschnitte 32 in dem rundbodenförmigen Randabschnitt 13 zusammenlaufen. Die freien Abschnitte 32 sind mit Bezug auf den Einführzustand in der Ebene des Basisteils 9 gegenüber den geradlinigen Befestigungsabschnitten 31 nach außen abgewinkelt. Der Ummantelungsteil 3 bildet mit Bezug auf den Einführzustand im Bereich zwischen den Befestigungsabschnitten 31 eine Aussparung zur Aufnahme und Befestigung des Basisteils 9. Durch die besondere Formgebung der inflatierbaren Randabschnitte 11, 12, 13 wird sichergestellt, daß der Ummantelungsteil 3 eine geringe Baugröße beim Einführen in den Körper aufweist, was die minimal invasive Einbringung ermöglicht. Darüber hinaus ist sichergestellt, daß es beim Inflatieren zur Ummantelung des Herzens 2 ausgehend von vorne nach hinten über die Seitenwände 7 und die Herzspitze 8 bis in den Bereich der Hinterwände des Herzens 2 kommt. Zwischen den freien Abschnitten 32 ist ein ebener Bereich 33 des Ummantelungsteils 3 vorgesehen, der nicht eingerollt bzw. gefaltet ist.

Grundsätzlich ist es natürlich auch möglich, daß der Basisteil 9 und der Ummantelungsteil 3 über die gesamte Länge des Basisteils 9 vollflächig miteinander verbunden sind, wobei der Basisteil 9 an die Wölbung des Ummantelungsteils 3 angepaßt ist.

In Fig. 5 ist dargestellt, daß die beiden längsseitigen inflatierbaren Randabschnitte 11, 12 von außen in Richtung zum Basisteil 9 hin eingerollt sind und daß die Schichtdicke der Randabschnitte 11, 12 im wesentlichen konstant ist. Dadurch wird ein gleichmäßiges Ausrollen der Randabschnitte 11, 12 beim Überführen der erfindungsgemäßen Vorrichtung 1 in den Funktionszustand gewährleistet. Die Randabschnitte 11, 12 gehen in Richtung zum Basisteil 9 trompetenartig in Befestigungsabschnitte 34a über, die Befestigungsflächen 34 aufweisen. Die Breite der Befestigungsflächen 34 entspricht dabei der Breite der Längsseitenflächen 35 des Basisteils 9, so daß eine sichere Befestigung der Randabschnitte 11, 12 an dem Basisteil 9 möglich ist.

Bei der dargestellten Ausführungsform sind die Randabschnitte 11, 12 im Bereich der geraden Befestigungsabschnitte 31 durch das Basisteil 9 voneinander getrennt und nicht unmittelbar miteinander verbunden. Grundsätzlich können die Randabschnitte 11, 12 in diesem Bereich des Ummantelungsteils 3 auch unmittelbar miteinander verbunden sein, wobei der Basisteil 9 von oben zwischen den Randabschnitten 11, 12 auf den Ummantelungsteil 3 aufgesetzt und mit dem Ummantelungsteil 3 befestigt sein kann.

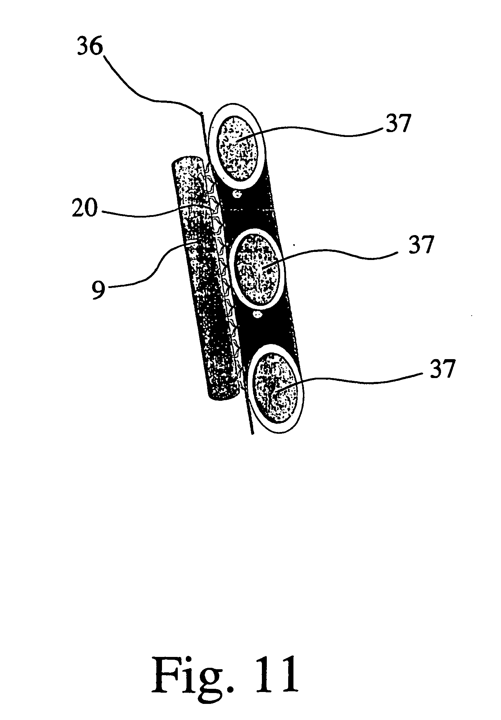

In Fig. 11 ist die Befestigung des Basisteils 9 mit den Saugnäpfen 20 an einer Innenwand 36 des Thorax schematisch dargestellt. Unterhalb der Innenwand 36 verlaufen die schematisch dargestellten Rippen 37. Nicht dargestellt ist der Ummantelungsteil 3, der mit dem Basisteil 9 an der Innenwand 36 gehalten ist.

Der Ummantelungsteil 3 erstreckt sich ausgehend von dem oberen Rand 25 des Basisteils 9 bis über die Unterkante 38 des Basisteils 9 in Richtung zur Herzspitze 8. Während des Einführvorgangs bzw. im Einführzustand ist der Ummantelungsteil 3 eingerollt (vgl. Fig. 4). Nach dem Einführen bzw. nach Plazieren der Vorrichtung 1 in der Brusthöhle wird der Ummantelungsteil 3 im Randbereich ausgerollt und umgreift somit das Herz 2 (vgl. Fig. 1).

Anhand der Fig. 7 und 8 sowie 12 und 13 wird nun der Aufbau des Ummantelungsteils 3 näher beschrieben. In Fig. 7 ist eine Querschnittsansicht der Vorrichtung 1 im Funktionszustand dargestellt. Der Ummantelungsteil 3 weist einen mehrschichtigen Aufbau auf und besteht aus inneren und äußeren Applikationsschichten 22, die in den Fig. 14a und 14b dargestellt sind. Die Applikationsschichten 22 erstrecken sich auf der Innenseite 39 und auf der Außenseite 40 der Vorrichtung 1 über die gesamte Fläche des Ummantelungsteils 3. Die Applikationsschichten 22 sind als innere bzw. äußere Grenzschicht des Ummantelungsteils 3 ausgebildet und weisen ein netzartiges Kanälchensystem mit porenartigen Öffnungen 23 zur Innenseite 39 und Außenseite 40 des Ummantelungsteils 3 auf. Das Kanälchensystem wird durch Zu- und Ableitungen gespeist. Hierzu sind ein oder mehrere druckstabile, gas- und flüssigkeitsdichte Fluidleitungen 41 vorgesehen, die zur Versorgung der Applikationsschichten 22 des Funktionsteils 3 und ggf. des Basisteils 9 ausgebildet sind. Damit können in das netzartige Kanälchensystem Flüssigkeiten, Gele, Gas oder Gasgemische eingeleitet bzw. abgeleitet werden. Durch die Applikation von Flüssigkeiten oder Gelen über die Kanälchensysteme auf die innere und/oder äußere Oberfläche des Ummantelungsteils 3 wird dieser entsprechend benetzt, es entsteht ein Flüssigkeitsfilm zwischen den Oberflächen des Ummantelungsteils 3 der Vorrichtung 1 und den angrenzenden Organen bzw. Grewebestrukturen (innen: Herz; außen: Lungen). Dadurch wird ein direkter Kontaktschluß zwischen den Flächen der Vorrichtung 1 vor allem mit dem Herzen 2 und den Lungen vermieden.

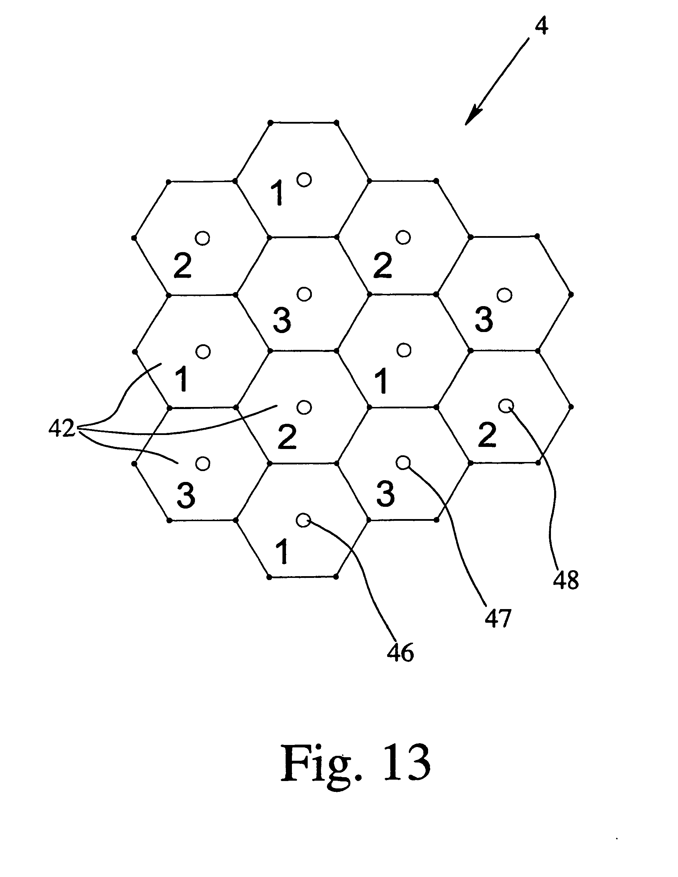

Darüber hinaus weist der Ummantelungsteil 3 ein oder mehrere Stabilisierungsschichten 4 auf. Die Stabilisierungsschicht 4 weist eine Wabenstruktur mit einer Mehrzahl von nebeneinanderliegenden inflatierbaren Wabenkammern 42 auf, wobei die Wabenkammern 42 mit Fluidleitungen 43 zur zyklischen Zufuhr eines Fluides in die Wabenkammern 42 verbunden sind. Die Fluidleitungen 43 sind sehr hochdruckstabil, gas- und flüssigkeitsdicht. Der wabenartige Aufbau der Stabilisierungsschicht 4 ist in Fig. 13 dargestellt. Die Wabenkammern 42 weisen einen konisch in Richtung zum Herzen 2 zulaufenden Querschnitt mit größerer Grundfläche auf der herzfernen Seite und mit kleinerer Grundfläche auf der herznahen Seite auf, so daß bei Zufuhr eines Fluides in die Wabenkammern 42 die Wabenkammern 42 deflatieren und eine nach innen gerichtete Wölbung des Ummantelungsteils 3 bewirkt wird. Nicht dargestellt ist, daß grundsätzlich auch mehrere einander bereichsweise überlappende Stabilisierungsschichten 4 vorgesehen sein können. Die Stabilisierungsschicht 4 erstreckt sich ebenfalls über die gesamte Fläche des Ummantelungsteils 3. Sie besteht aus schwer oder gar nicht dehnbaren und flexiblen Materialien.

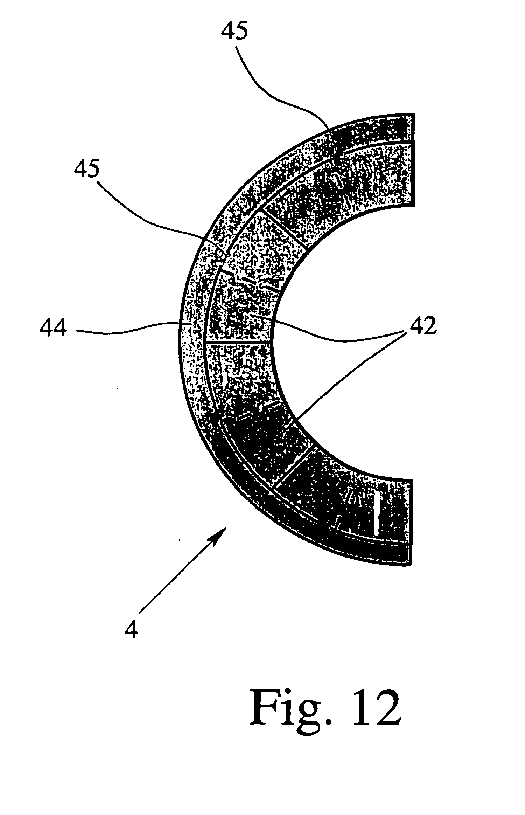

Wie sich aus Fig. 12 ergibt, ist die Stabilisierungsschicht 4 ebenfalls mehrschichtig aufgebaut und weist neben den Wabenkammern 42 eine äußere Versorgungsschicht 44 auf, die über Zu- bzw. Ableitungen verfügt, über welche Gase, Gasgemische oder Flüssigkeiten den Wabenkammern 42 zugeführt bzw. aus den Wabenkammern 42 abgeführt werden können. Dazu sind in der Versorgungsschicht 44 Öffnungen 45 vorgesehen, die einen Übertritt von Fluiden aus der Versorgungsschicht 44 in die Wabenkammern 42 ermöglichen. Im Einführzustand sind die Wabenkammern 42 zusammengefaltet bzw. zusammengelegt. Beim Einbringen eines komprimierten Gases oder Gasgemisches über die Versorgungsschicht 44 in die schwer oder nicht dehnbaren Wabenkammern 42 werden diese entfaltet und verleihen damit dem Ummantelungsteil 3 der Vorrichtung 1 die das Herz 2 umgreifende Form. Dadurch, daß der hohe Druck in dem schwer oder nicht dehnbaren Wabensystem aufrecht erhalten wird, erhält das Ummantelungssystem seine Stabilität bzw. notwendige Steifigkeit in der Brusthöhle. Dazu ist die Stabilisierungsschicht 4 bestehend aus dem wabenartigen Kammersystem und der Versorgungsschicht 44 sowie den Fluidleitungen 43 aus entsprechen gewählten gas- und druckdichten sowie schwer bzw. nicht dehnbaren Materialien aufgebaut.

Zur Versorgung der Wabenkammern 42 mit einem komprimierten Gas oder Gasgemisch sind voneinander getrennte Zugänge 46, 47, 48 in benachbarten Wabenkammern 42 vorgesehen. Drei benachbarte Wabenkammern 42 (in Fig. 13 mit unterschiedlichen eingeschriebenen Zahlen 1 bis 3 gekennzeichnet) sind dabei durch unterschiedliche Fluidleitungen 43 befüllbar. Es werden somit nicht benachbarte Wabenkammern 42 über eine gemeinsame Zuleitung versorgt. Würde ein Leck beispielsweise in einer Wabenkammer 42 vom Typ 1 entstehen, ist keine direkt benachbarte Wabenkammer 42 vom Typ 2 oder 3 betroffen. Der Ummantelungsteil 3 der Vorrichtung 1 kann dann durch die Wabenkammern 42 des Typs 2 und 3 die Steifigkeit und Stabilität für die Vorrichtung 1 aufrechterhalten.

Schließlich weist der Ummantelungsteil 3 der Vorrichtung 1 wenigstens eine Augmentationsschicht 5 auf, die sich im Gegensatz zu den Applikationsschichten 22 und der Stabilisierungsschicht 4 innerhalb des Ummantelungsteils 3 nur im Bereich über den Seitenwänden 7 und den Hinterwänden der Herzkammern und nicht, wie die anderen Schichten, über die gesamte Ausdehnung des Ummantelungsteils 3 erstreckt. Die Augmentationsschicht 5 besteht aus einem dehnbaren Material. Über eine oder mehrere Fluidleitungen 49, die druckstabil, gas- und flüssigkeitsdicht ausgebildet sind, kann ein Gas oder Gasgemisch zyklisch in den Bereich zwischen der Stabilisierungsschicht 4 und der Augmentationsschicht 5 eingefüllt werden, so daß sich die Augmentationsschicht 5 aufbläht, was in Fig. 7 dargestellt ist. Dadurch, daß die der Augmentationsschicht 5 außen anliegende Stabilisierungsschicht 4 schwer oder gar nicht dehnbar ist, erfolgt ein gerichtetes Aufblähen der Augmentationsschicht 5 nach innen in Richtung der Herzkammern. Die Herzkammern werden durch das zyklische Pumpen und in der Folge zyklische Aufblähen der dehnbaren Augmentationsschicht 5 entsprechend komprimiert und dekomprimiert, was zu einer Unterstützung der Pumpfunktion der Herzkammern in der Art einer Herzdruckmassage führt.

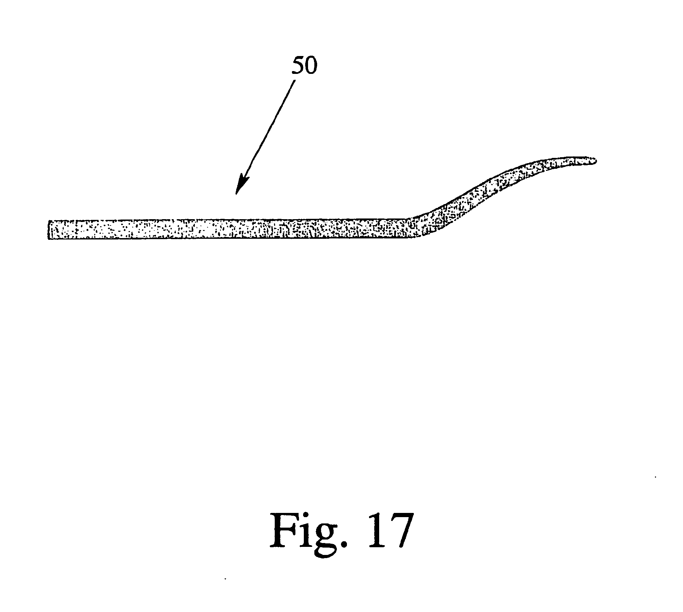

In Fig. 17 ist eine spatelartige Sondierungshilfseinrichtung 50 dargestellt, die zur Sondierung des Herzens 2 vorgesehen ist, nachdem durch einen kleinen Hautschnitt von ca. 3 bis 5 cm ein Zugang im Bereich zwischen dem Schwertfortsatz des Brustbeins und dem Unterrand des linken Rippenbogens zur Bauchhöhle geschaffen wurde. Die Sondierungshilfseinrichtung 50 wird dazu durch die geschaffene Öffnung in die Bauchhöhle eingeführt, nach oben in Richtung der Brusthöhle vorgeschoben, gelangt durch den Lareyischen Spalt des Zwerchfells in die Brusthöhle und wird von dort weiter in Richtung des Herzens 2 geführt. Das Herz 2 wird sondiert und an der Vorderseite des Herzens 2 mit der Sondierungshilfseinrichtung 50 umfahren. Dabei werden eventuelle Verwachsungen des Herzbeutels mit der inneren Brustwand stumpf abgelöst.

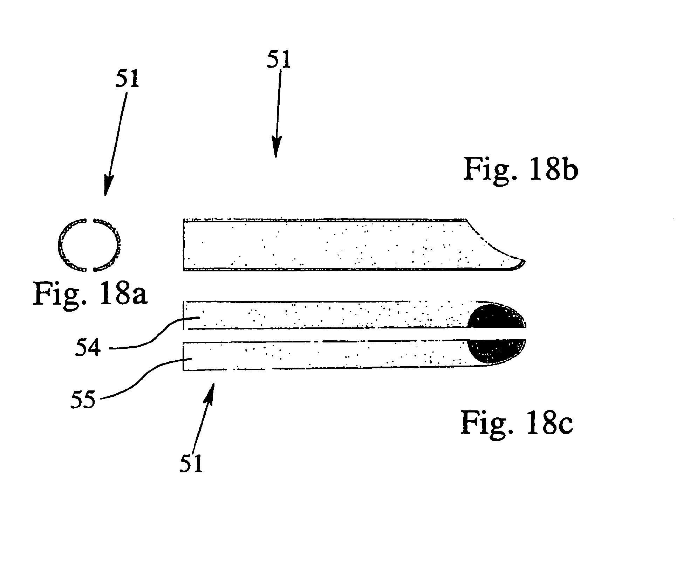

Nach der Sondierung mit der Sondierungshilfseinrichtung 50 und deren Entfernung aus der Brust- und der Bauchhöhle wird eine rohrartige Einführeinrichtung 51, die in den Fig. 18a bis 18c dargestellt ist, über den gleichen Zugangsweg zum Herzen 2 geführt. Nach Erreichen der Herzspitze 8 kann die Vorrichtung 1 mittels einer beweglichen Positionierungseinrichtung 52 durch einen rohrartigen Einführungskanal der Einführeinrichtung 51 bis zum Herzen 2 vorgeschoben werden. Bei Erreichen der Herzspitze 8 wird die Einführeinrichtung 51 leicht zurückgezogen, damit ein Gelenk 53 der Positionierungseinrichtung 52 aus dem proximalen, im Körper befindlichen Ende der Einführeinrichtung 51 freigegeben wird. Jetzt kann die am Gelenk 53 befestigte Vorrichtung 1 zwischen der Herzvorderwand und Innenwand 36 des Brustkorbes in Position gebracht werden. Ist die Vorrichtung 1 in Position gebracht mit einer Ausrichtung zur Herzachse X wird die Vorrichtung 1 über die Saugvorrichtung bzw. mittels der am Basisteil 9 vorgesehenen Saugnäpfe 20 an der Innenwand 36 des Brustkorbes befestigt. Jetzt kann die Positionierungseinrichtung 52 vom Basisteil 9 der Vorrichtung 1 abgetrennt werden und durch den Kanal der Einführeinrichtung 51 aus dem Körperinneren herausgezogen werden. Danach wird die Einführeinrichtung 51 aus dem Körper entfernt. Außerhalb des Körpers können dann zwei lösbar miteinander verbundene Halbteile 54, 55 der Einführeinrichtung 51 voneinander getrennt werden, um die Einführeinrichtung 51 von den Leitungen 21, 41, 43 und 49 zu trennen.

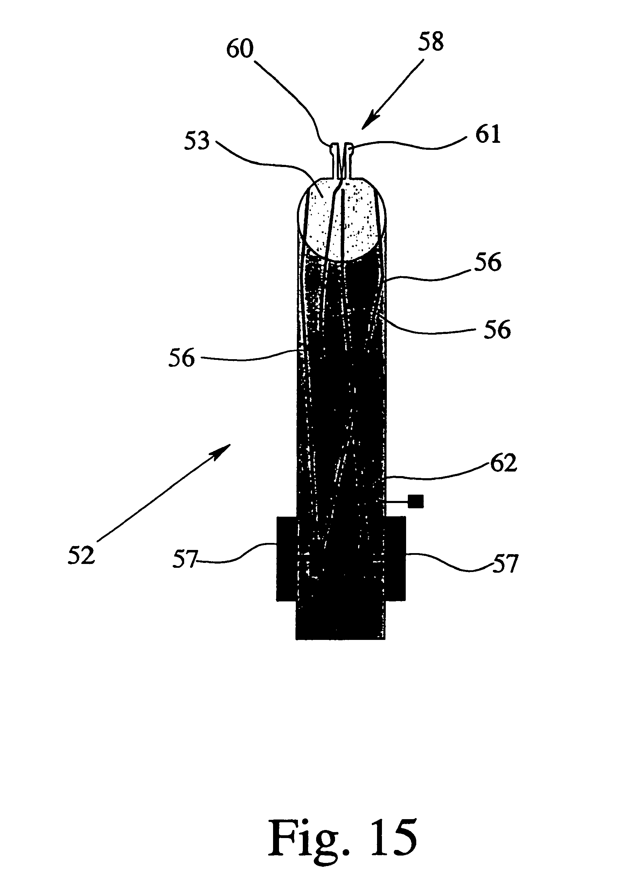

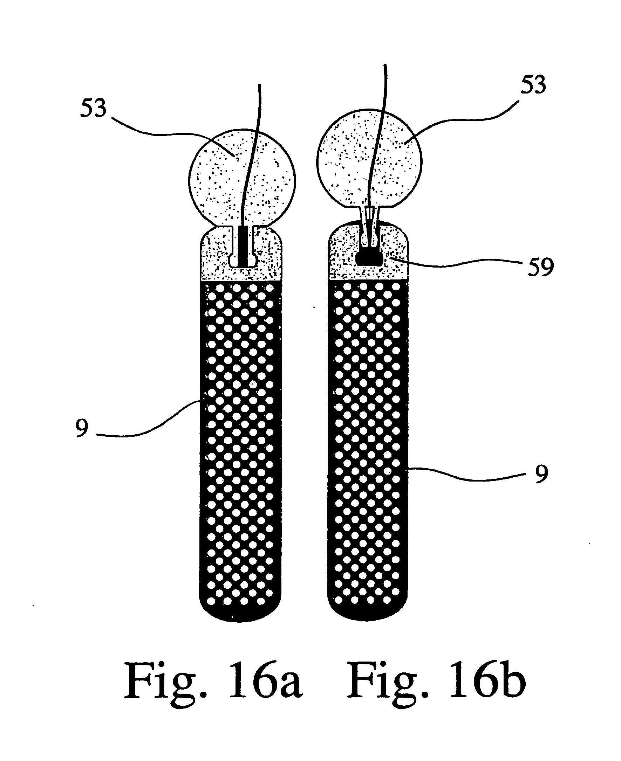

In Fig. 15 ist die Positionierungseinrichtung 52 in einer schematischen Querschnittsansicht dargestellt. Das Gelenk 53 läßt sich über Drähte 56 und Stellschrauben 57 in die gewünschte Richtung drehen. Am Kopf des Gelenks 53 ist ein Befestigungsabschnitt 58 vorgesehen, der mit einem komplementär ausgebildeten Befestigungsabschnitt 59 am Basisteil 9 eine Rastverbindung ausbilden kann. Der Befestigungsabschnitt 59 ist an dem oberen Rand 25 des Basisteils 9 vorgesehen. Dies ist in den Fig. 16a und 16b gezeigt. Der Befestigungsabschnitt 58 weist zwei Befestigungsschenkel 60, 61 auf, die über einen Steuerungsdraht 62 zusammengezogen werden können, um die gebildete Rastverbindung zu lösen.

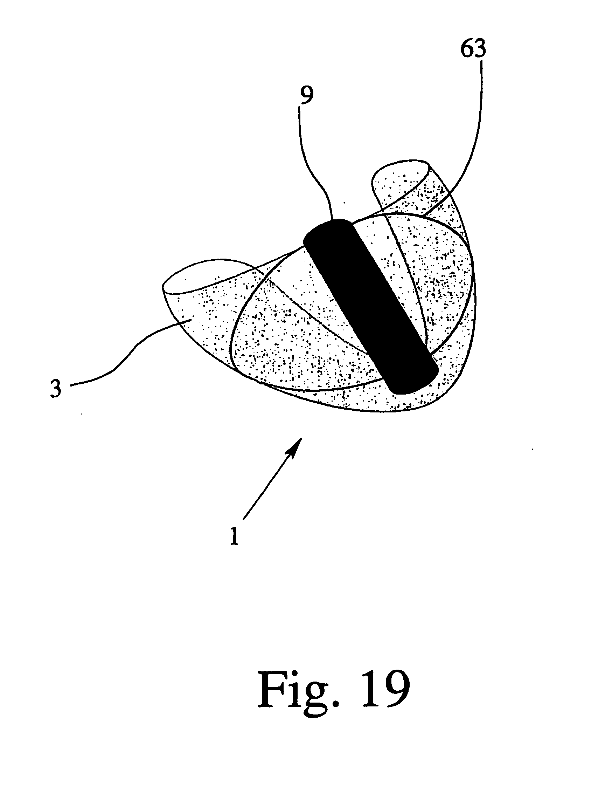

In Fig. 19 ist dargestellt, daß ein Stabilisierungsdraht 63 für den Ummantelungsteil 3 vorgesehen sein kann, wobei der Stabilisierungsdraht 63 zwischen der Stabilisierungsschicht 4 und der Augmentationsschicht 5 verlaufend angeordnet ist.

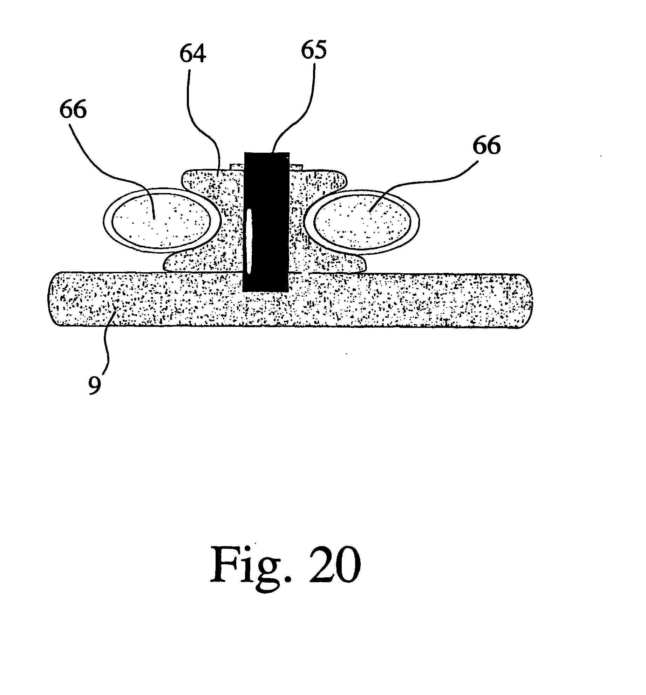

In Fig. 20 ist dargestellt, daß sich das Basisteil 9 und damit die Vorrichtung 1 über einen Gelpack 64 mit einem Befestigungselement 65 auch an Rippen 66 des Brustkorbes befestigen läßt.

## Patentansprüche

1. Vorrichtung (1) zur Unterstützung und/oder Übernahme der Pumpfunktion des Herzens (2), mit einem mehrschichtigen Ummantelungsteil (3) ausgebildet zur zumindest bereichsweisen Kompression des Herzens (2), wobei der Ummantelungsteil (3) wenigstens eine Stabilisierungsschicht (4) zur Formgebung des Ummantelungsteils (3), wenigstens eine innere dehnbare Augmentationsschicht (5) aufweist und wobei zwischen der Stabilisierungsschicht (4) und der Augmentationsschicht (5) wenigstens ein mittels eines Fluides inflatierbarer und deflatierbarer Hohlraum (6) für die zyklische Kompression des Herzens (2) gebildet wird, wobei die Stabilisierungsschicht (4) mittels eines Fluides zumindest bereichsweise inflatierbar ist, um den Ummantelungsteil (3) von einem zusammengerollten und/oder zusammengefalteten Einführzustand beim Einführen der Vorrichtung in einen Körper in einen gewölbten Funktionszustand zu überführen, und wobei das Herz (2) im Funktionszustand von dem Ummantelungsteil (3) zumindest teilweise ummantelt und/oder umgriffen wird **dadurch gekennzeichnet, daß** der Ummantelungsteil (3) mit Bezug auf den Einführzustand zwei sich in Längsrichtung erstreckende aufgerollte und/oder gefaltete inflatierbare Randabschnitte (11, 12) aufweist, wobei ein Basisteil (9) zwischen den Randabschnitten (11, 12) angeordnet ist und wobei die beiden Randabschnitte (11, 12) in einem gemeinsamen inflatierbaren rundbodenförmigen Randabschnitt (13) zum Ummanteln und/oder Umgreifen der Herzspitze (8) zusammenlaufen.

2. Vorrichtung nach Anspruch 1, **dadurch gekennzeichnet, daß** jeder sich in Längsrichtung erstreckende aufgerollte und/oder gefaltete Randabschnitt (11, 12) mit Bezug auf den Einführzustand einen geradlinigen Befestigungsabschnitt (31) zur Befestigung mit dem Basisteil (9) und einen an den geradlinigen Befestigungsabschnitt (31) anschließenden aus der Ebene des Basisteils (9) herausgebogenen nicht an dem Basisteil (9) befestigten freien Abschnitt (32) aufweist, wobei die auf beiden Längsseiten des Basisteils (9) angeordneten freien Abschnitte (32) in dem rundbodenförmigen Randabschnitt (13) zusammenlaufen.

3. Vorrichtung nach Anspruch 2, **dadurch gekennzeichnet, daß** die freien Abschnitte (32) mit Bezug auf den Einführzustand in der Ebene des Basisteils (9) gegenüber den geradlinigen Befestigungsabschnitten (31) nach außen abgewinkelt und/oder abgebogen sind.

4. Vorrichtung nach Anspruch 2 oder 3, **dadurch gekennzeichnet, daß** der Ummantelungsteil (3) mit Bezug auf den Einführzustand im Bereich zwischen den Befestigungsabschnitten (31) eine Aussparung (18) zur Aufnahme und Befestigung des Basisteils (9) aufweist.

5. Vorrichtung nach einem der vorhergehenden Ansprüche, **dadurch gekennzeichnet, daß** der Ummantelungsteil (3) im Funktionszustand eine an die Form des Herzens (2) angepaßte kelchartige Tulpenform aufweist, so daß das Herz (2) im Funktionszustand von dem Ummantelungsteil (3) von vorne nach hinten über die Seitenwände (7) des Herzens (2) und, vorzugsweise, über die Herzspitze (8) bis in den Bereich der Hinterwände des Herzens (2) ummantelt und/oder umgriffen wird.

6. Vorrichtung nach einem der vorhergehenden Ansprüche, **dadurch gekennzeichnet, daß** der Ummantelungsteil (3) im Einführzustand einen von außen nach innen spiralförmig aufgerollten und/oder gefalteten inflatierbaren Randabschnitt (11, 12, 13) aufweist.

7. Vorrichtung nach einem der vorhergehenden Ansprüche, **dadurch gekennzeichnet, daß** der Ummantelungsteil (3) auf der mit Bezug auf die Anordnung im Funktionszustand der Hinterwand des Herzens (2) zugewandeten Hinterseite (17) eine nach oben geöffnete vorzugsweise hyperbelförmige Aussparung (18) für die großen Herzgefäße aufweist.

8. Vorrichtung nach einem der vorhergehenden Ansprüche, **dadurch gekennzeichnet, daß** der Ummantelungsteil (3) das Herz (2) im Funktionszustand fingerartig umgreift.

9. Vorrichtung nach einem der vorhergehenden Ansprüche, **dadurch gekennzeichnet, daß** wenigstens ein Stabilisierungsdraht (63) für den Ummantelungsteil (3) vorgesehen ist, wobei, vorzugsweise, der Stabilisierungsdraht (63) zwischen der Stabilisierungsschicht (4) und der Augmentationsschicht (5) angeordnet ist.

10. System zur Unterstützung und/oder Übernahme der Pumpfunktion des Herzens (2) mit einer Vorrichtung (1) nach einem der vorhergehenden Ansprüche und mit einer stab- und/oder rohrförmigen Positionierungseinrichtung (52), wobei die Positionierungseinrichtung (52) einen beweglichen Kopfabschnitt und Steuerungsmittel zur Steuerung der Bewegung des Kopfabschnittes aufweist und wobei an dem Kepfabschnitt wenigstens ein Befestigungsabschnitt (58) zur lösbaren Befestigung mit dem Befestigungsabschnitt (59) des Basisteils (9) vorgesehen ist.

11. System zur Unterstützung und/oder Übernahme der Pumpfunktion des Herzens (2) nach Anspruch 10, mit einer Einführeinrichtung (51) zum Einführen der Vorrichtung (1) in den Körper, wobei die Einführeinrichtung (51) einen Einführkanal für die Vorrichtung (1) und die Positionierungseinrichtung (52) aufweist.

12. System zur Unterstützung und/oder Übernahme der Pumpfunktion des Herzens (2) mit einer Vorrichtung (1) nach einem der vorhergehenden Ansprüche 1 bis 9, insbesondere System nach Anspruch 10 oder 11, mit einer spatelartigen Sondierungseinrichtung (50), wobei die Sondierungseinrichtung (50) zur Sondierung des Herzens (2) und zum stumpfen Ablösen von Verwachsungen des Herzbeutels mit der inneren Brustwand ausgebildet ist.

## Claims

1. A device (1) for supporting and/or performing the pumping function of the heart (2), having a multi-layer sheathing part (3) configured for at least locally compressing the heart (2), wherein the sheathing part (3) has at least one stabilizing layer (4) for shaping the sheathing part (3), at least one internal extensible augmentation layer (5), and wherein between the stabilization layer (4) and the augmentation layer (5), at least one cavity (6), which is inflatable and deflatable by means of a fluid, is formed for cyclic compression of the heart (2), wherein the stabilization layer (4) is inflatable at least locally by means of a fluid so as to transition the sheathing part (3) from a coiled and/or folded insertion state when the device is inserted into a body into a curved operational state, and wherein the heart (2) in the operational state is at least partially sheathed and/or surrounded by the sheathing part (3), **characterized in that** the sheathing part (3) has two coiled and/or folded inflatable edge portions (11, 12) extending in the longitudinal direction in relation to the insertion state, wherein a base part (9) is arranged between the edge portions (11, 12), and wherein the two edge portions (11, 12) converge into a common inflatable edge portion (13) shaped as a round bottom for sheathing and/or surrounding the cardiac apex (8).

2. The device according to claim 1, **characterized in that** each coiled and/or folded edge portion (11, 12) extending in the longitudinal direction has in relation to the insertion state a linear fastening portion (31) for fastening to the base part (9), and a free portion (32) following the linear fastening portion (31), bent out of the plane of the base part (9) and not fastened to the base part (9), wherein the free portions (32) arranged on both longitudinal sides of the base part (9) converge into the edge portion (13) shaped as a round bottom.

3. The device according to claim 2, **characterized in that** in relation to the insertion state, the free portions (32) are angled and/or bent outward in the plane of the base part (9) with respect to the linear fastening portions (31).

4. The device according to claim 2 or 3, **characterized in that** in relation to the insertion state, the sheathing part (3) in the area between the fastening portions (31) has a recess (18) for receiving and fastening the base part (9).

5. The device according to any of the preceding claims, **characterized in that** in the operational state, the sheathing part (3) has a cup-like tulip shape adapted to the shape of the heart (2), so that in the operational state, the heart (2) is sheathed and/or surrounded by the sheathing part (3) from front to back via the side walls (7) of the heart (2), and preferably via the cardiac apex (8) up to the area of the back walls of the heart (2).

6. The device according to any of the preceding claims, **characterized in that** in the insertion state, the sheathing part (3) has an inflatable edge portion (11, 12, 13) spirally coiled and/or folded from outside to inside.

7. The device according to any of the preceding claims, **characterized in that** the sheathing part (3), at the rear (17) oriented toward the rear wall of the heart (2) in relation to the arrangement in the operational state has a recess (18) open at the top and preferably of hyperbolic shape for the large coronary vessels.

8. The device according to any of the preceding claims, **characterized in that** in the operational state, the sheathing part (3) surrounds the heart (2) like fingers.

9. The device according to any of the preceding claims, **characterized in that** at least one stabilization wire (63) is provided for the sheathing part (3), wherein preferably the stabilization wire (63) is arranged between the stabilization layer (4) and the augmentation layer (5).

10. A system for supporting and/or performing the pumping function of the heart (2), comprising a device (1) according to any of the preceding claims and a rod and/or tube-shaped positioning device (52), wherein the positioning device (52) has a movable head portion and control means for controlling movement of the head portion, and wherein on the head portion, at least one fastening portion (58) is provided for releasable fastening to the fastening portion (59) of the base part (9).

11. The system for supporting and/or performing the pumping function of the heart (2) according to claim 10, comprising an insertion device (51) for inserting the device (1) into the body, wherein the insertion device (51) has an insertion passage for the device (1) and the positioning device (52).

12. The system for supporting and/or performing the pumping function of the heart (2), comprising a device (1) according to any of the preceding claims 1 to 9, in particular the system according to claim 10 or 11, with a spatulate probe device (50), wherein the probe device (50) is made for probing the heart (2) and for blunt separation of concrescences of the pericardium with the internal chest wall.

## Revendications

1. Dispositif (1) pour prendre en charge et/ou effectuer la fonction de pompage du coeur (2), comprenant une partie de gaine multicouche (3) configurée pour comprimer le coeur (2) au moins par endroits, dans lequel la partie de gaine (3) présente au moins une couche de stabilisation (4) pour la conformation de la partie de gaine (3), au moins une couche d'augmentation (5) extensible intérieure, et dans lequel entre la couche de stabilisation (4) et la couche d'augmentation (5) est formée au moins une cavité (6) gonflable et dégonflable au moyen d'un fluide pour la compression cyclique du coeur (2), dans lequel la couche de stabilisation (4) est gonflable au moins par endroits au moyen d'un fluide de façon à faire passer la partie de gaine (3) d'un état d'insertion enroulé et/ou replié lorsque le dispositif est inséré dans un corps à un état de fonctionnement bombé, et dans lequel à l'état de fonctionnement, le coeur (2) est au moins partiellement gainé et/ou entouré par la partie de gaine (3), **caractérisé en ce que** la partie de gaine (3) présente deux portions de bord gonflables (11, 12), enroulées et/ou repliées, s'étendant dans la direction longitudinale par rapport à l'état d'insertion, dans lequel une partie de base (9) est agencée entre les portions de bord (11, 12), et dans lequel les deux portions de bord (11, 12) convergent vers une portion de bord gonflable (13) en forme de fond arrondi pour gainer et/ou entourer le sommet du coeur (8).

2. Dispositif selon la revendication 1, **caractérisé en ce que** chaque portion de bord enroulée et/ou repliée (11, 12) s'étendant dans la direction longitudinale présente par rapport à l'état d'insertion une portion de fixation rectiligne (31) pour la fixation à la partie de base (9), et une portion libre (32) suivant la portion de fixation rectiligne (31), dépliée hors du plan de la partie de base (9) et non fixée à la partie de base (9), dans lequel les portions libres (32) agencées sur les deux côtés longitudinaux de la partie de base (9) convergent dans la portion de bord (13) en forme de fond arrondi.

3. Dispositif selon la revendication 2, **caractérisé en ce que** par rapport à l'état d'insertion, les portions libres (32) sont coudées et/ou écartées vers l'extérieur dans le plan de la partie de base (9) par rapport aux portions de fixation rectilignes (31).

4. Dispositif selon la revendication 2 ou 3, **caractérisé en ce que** par rapport à l'état d'insertion, la partie de gaine (3) présente dans la zone entre les portions de fixation (31) un creux (18) pour recevoir et fixer la partie de base (9).

5. Dispositif selon l'une quelconque des revendications précédentes, **caractérisé en ce qu'**à l'état de fonctionnement, la partie de gaine (3) présente une forme de tulipe à la manière d'une coupe, adaptée à la forme du coeur (2), de sorte qu'à l'état de fonctionnement, le coeur (2) est gainé et/ou entouré par la partie de gaine (3) d'avant en arrière par l'intermédiaire des parois latérales (7) du coeur (2), et de préférence par l'intermédiaire du sommet du coeur (8) jusque dans la zone des parois arrière du coeur (2).

6. Dispositif selon l'une quelconque des revendications précédentes, **caractérisé en ce qu'**à l'état d'insertion, la partie de gaine (3) comprend une portion de bord gonflable (11, 12, 13) enroulée et/ou repliée en spirale de l'extérieur vers l'intérieur.

7. Dispositif selon l'une quelconque des revendications précédentes, **caractérisé en ce que** la partie de gaine (3), du côté arrière (17), orienté vers la paroi arrière du coeur (2) par rapport à l'agencement à l'état de fonctionnement, présente un creux (18) ouvert vers le haut et de préférence de forme hyperbolique pour les grands vaisseaux coronaires.

8. Dispositif selon l'une quelconque des revendications précédentes, **caractérisé en ce qu'**à l'état de fonctionnement, la partie de gaine (3) entoure le coeur (2) comme des doigts.

9. Dispositif selon l'une quelconque des revendications précédentes, **caractérisé en ce qu'**au moins un fil de stabilisation (63) est fourni pour la partie de gaine (3), dans lequel le fil de stabilisation (63) est de préférence agencé entre la couche de stabilisation (4) et la couche d'augmentation (5).

10. Système pour prendre en charge et/ou effectuer la fonction de pompage du coeur (2), comprenant un dispositif (1) selon l'une quelconque des revendications précédentes et un dispositif de positionnement (52) en forme de tige et/ou de tube, dans lequel le dispositif de positionnement (52) présente une portion de tête mobile et des moyens de commande pour commander le déplacement de la portion de tête, et dans lequel sur la portion de tête, au moins une portion de fixation (58) est fournie pour la fixation amovible à la portion de fixation (59) de la partie de base (9).

11. Système pour prendre en charge et/ou effectuer la fonction de pompage du coeur (2) selon la revendication 10, comprenant un dispositif d'insertion (51) pour insérer le dispositif (1) dans le corps, dans lequel le dispositif d'insertion (51) présente un passage d'insertion pour le dispositif (1) et le dispositif de positionnement (52).

12. Système pour prendre en charge et/ou effectuer la fonction de pompage du coeur (2), comprenant un dispositif (1) selon l'une quelconque des revendications précédentes 1 à 9, en particulier un système selon la revendication 10 ou 11, avec un dispositif de sondage (50) de type spatule, dans lequel le dispositif de sondage (50) est réalisé pour sonder le coeur (2) et pour séparer par clivage des concrescences du péricarde avec la paroi thoracique intérieure.
